(19) Europäisches Patentamt
European Patent Office
Office européen des brevets

(11) EP 2 024 488 B1

(12) # EUROPEAN PATENT SPECIFICATION

(45) Date of publication and mention of the grant of the patent:
**17.01.2018 Bulletin 2018/03**

(21) Application number: **07756908.5**

(22) Date of filing: **13.02.2007**

(51) Int Cl.:
***G01N 21/76*** *(2006.01)* ***G01N 21/55*** *(2014.01)*
***G01N 33/553*** *(2006.01)* *G01N 21/552* *(2014.01)*

(86) International application number:
**PCT/US2007/062041**

(87) International publication number:
**WO 2007/095527 (23.08.2007 Gazette 2007/34)**

(54) **METAL-ENHANCED CHEMILUMINESCENCE (MEC)**

METALLVERBESSERTE CHEMILUMINESZENZ (MEC)

CHIMIOLUMINESCENCE AMÉLIORÉE PAR LES MÉTAUX (MEC)

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.02.2006 US 773037 P**

(43) Date of publication of application:
**18.02.2009 Bulletin 2009/08**

(73) Proprietor: **Geddes, Chris**
**Bel Air, MD 21015 (US)**

(72) Inventor: **Geddes, Chris**
**Bel Air, MD 21015 (US)**

(74) Representative: **HGF Limited**
**Saviour House**
**9 St. Saviourgate**
**York YO1 8NQ (GB)**

(56) References cited:
**US-A- 4 672 040**
**US-A- 5 779 976**
**US-A- 6 066 448**
**US-A1- 2003 082 633**
**US-B1- 6 362 011**

- **JIAN ZHANG ET AL: "First observation of surface plasmon-coupled electrochemiluminescence", CHEMICAL PHYSICS LETTERS, vol. 393, no. 4-6, 1 August 2004 (2004-08-01), pages 483-487, XP055048062, ISSN: 0009-2614, DOI: 10.1016/j.cplett.2004.06.050**
- **KADIR ASLAN ET AL: "Microwave-Accelerated Metal-Enhanced Fluorescence: Platform Technology for Ultrafast and Ultrabright Assays", ANALYTICAL CHEMISTRY, vol. 77, no. 24, 1 December 2005 (2005-12-01), pages 8057-8067, XP055048061, ISSN: 0003-2700, DOI: 10.1021/ac0516077**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 024 488 B1

## Description

BACKGROUND OF THE INVENTION

Field of the Invention

The present invention relates to bioassays, and more particularly, to the use of metallized surfaces to enhance intensity of chemiluminescence species or reactions in chemiluminescence assays thereby increasing sensitivity and detectability of same.

Background of the Related Art

The use of light-producing chemical reactions for quantitative detection in biotechnology is increasing [1-7], especially with regard to chemiluminescence based ligand-binding assays [1-7]. The attractiveness of chemiluminescence as an analytical tool lies primarily in the simplicity of detection [8]; the fact that most samples have no unwanted background luminescence, as is typically observed in fluorescence-based assays [9]; and the fact that no optical filters are required to separate the excitation wavelengths and scatter [8], as is also required for fluorescence-based detection [9].

However, chemiluminescent based detection is currently limited by the availability of chemiluminescent probes, which is not a factor governing fluorescence based detection [9]. Both fluorescence and chemiluminescence based technologies do however suffer from an inherent need for increased sensitivity/detection limits [8, 9]. For fluorescence, this is governed by the quantum yield of the tagging fluorophore, the level of unwanted background fluorescence and the photostability of the fluorophore [9], where as for chemiluminescence, detection is limited by the quantum efficiency of the chemiluminescence reaction or probe, and the time before depletion of the reactants [8]. For both detection systems, an increased luminescence yield would clearly benefit overall detectability and therefore for bioassays, the sensitivity towards a particular analyte.

Recent developments have provided new technology to enhance fluorescence and that can increase the system quantum yield [10-13], the photostability of the fluorophore [10-13] and by using spatially localized excitation can readily remove unwanted background fluorescence [14]. Specifically, techniques such as Metal-Enhanced Fluorescence (MEF) [10-20] also called Radiative Decay Engineering [21] and Surface Enhanced fluorescence (SEF) [22], have used nanosecond decay time fluorophores in close proximity to a variety of different sized [15] and shape [16,17] noble metal nanostructures to overcome the shortcomings of fluorescence technique. However, to date no one has found any comparable systems to overcome the shortcomings of using chemiluminescent based reaction detection methods.

US-A-2003/0082633 discloses a means for carrying out chemical preparations wherein reactions can be accelerated on special chips using microwave energy. The chips contain materials that efficiently absorb microwave energy, thereby causing chemical reaction rate increases and the method finds application in protein chemistry and combinatorial chemistry.

Jian Zhang et al, First Observation of Surface Plasmon-Coupled Electrochemiluminescence", Chemical Physics Letters, Vol. 393, No. 4-6, 1 August 2004, Pages 483-487, discuss electrochemiluminescence (ECL), which is often used for high sensitivity detection, and describe a new approach to collecting the ECL signal, by coupling of the excited state of $[Ru(bpy)_3]^{2+}$ with the surface plasmons in a thin gold film, such that the energy then radiates into a substrate at a defined angle. The authors suggest that the technique may be useful in chemical and biological assays.

SUMMARY OF THE INVENTION

The present invention is defined by the scope of the claims and any information that does not fall within the claims is provided for information only.

The present invention relates to surface plasmon-coupled chemiluminescence (SPCC), where the luminescence from chemically induced electronic excited states couple to surface plasmons in metallized particles or surfaces. Importantly, these plasmonic emissions emitted from a metallic particle or surface are generated without an external excitation source but instead from chemically induced electronically excited states.

The present invention relates to a method for measuring concentration of receptor-ligand binding complex in a test sample, the method comprising:

providing a metallic surface having positioned thereon a receptor molecule having affinity for a ligand of interest, wherein the metallic surface comprises metallic islands, metallic nanostructures, or metallic colloids;
contacting the receptor molecule with the test sample suspected of comprising the ligand of interest, wherein the ligand of interest will bind to the receptor molecule to form a receptor-ligand complex;

contacting the receptor-ligand complex with a detector molecule having affinity for the ligand to form a receptor-ligand-detector complex, wherein the detector molecule comprises a chemiluminescent label, wherein the chemiluminescent label is positioned about 5 nm to about 200 nm from the metallic surfaces;
triggering the chemiluminescent label to induce a chemically electronically excited state to produce chemiluminescence that couples with metallic surface plasmons for excitement thereof;
irradiating at least the test sample with microwave energy, wherein the microwave energy has a power input of from about 100 to 150 watts; and
measuring the intensity of radiation emitted from at least the excited metallic surface plasmons.

The present disclosure relates to bioassay systems comprising metallic surfaces for the enhancement of effects of chemiluminescence based reactions positioned near the metallic surfaces, wherein metallic surface plasmons are excited by a chemically induced electronically excited state of a chemiluminescent species and radiation emitted therefrom providing an enhanced signal.

The present disclosure relates to a bioassay for measuring concentration of receptor-ligand binding in a test sample, the method comprising:

(a) preparing metallic structures immobilized on a surface wherein the metallic structures have positioned thereon a receptor molecule having affinity for a ligand of interest;
(b) contacting the receptor molecule with the test sample suspected of comprising the ligand of interest, wherein the ligand of interest will bind to the receptor molecule to form a receptor-ligand complex;
(c) contacting the receptor-ligand complex with a detector molecule having affinity for the ligand to form a receptor-ligand-detector complex, wherein the detector molecule comprises a chemiluminescent label;
(d) exposing the chemiluminescent label to a trigger solution that will chemically react with the chemiluninescent label metal complex to induce a chemically electronically excited state; and
(e) measuring the intensity of radiation emitted from exited metallic surface plasmons.

The metallic surfaces comprise metallic islands, metallic nanostructures, or metallic colloids. The metallic element may include any form that exhibits surface plasmons such as noble metals including silver, gold, platinum and copper, and more preferably the metallic material is silver or gold.

The present disclosure relates to a method of metal-enhanced chemiluminescence sensing, comprising:

(a) applying a metallic material to a surface or within such surface used in a detection system;
(b) introducing a solution containing at least one biomolecule for disposing near the metallic surface, wherein the biomolecule comprises a chemiluminescent label;
(c) triggering the chemiluminescent label to induce a chemically electronically excited state thereby generating metallic surface plasmons; and
(d) measuring the chemiluminescence signal.

The present disclosure provides a method for detecting a targeted pathogen in a sample, the method comprising:

a) providing a system comprising:

i) a metallic surface, wherein the metallic surface has attached thereto an immobilized capture nucleic acid sequence probe complementary to a known nucleic acid sequence of the target pathogen; and
ii) a free capture nucleic acid sequence probe complementary to the known nucleic acid sequence of the target pathogen, wherein the free capture nucleic acid sequence probe has attached thereto a chemiluminescent label;

b) contacting the sample with the immobilized capture nucleic acid sequence probe, wherein the nucleic acid sequence of the target pathogen binds to the immobilized capture nucleic acid sequence probe;
c) contacting the bound nucleic acid sequence of the target pathogen with the free capture nucleic acid sequence probe for binding therewith;
d) introducing a trigger component to chemically react with the chemiluminescent label thereby creating a chemically induce electronically excited state that induces excited metallic surface plasmons; and
e) measuring the chemiluminescence signal intensity, wherein the signal is enhanced relative to system that does not include metallic surfaces.

The surface plasmon-coupled chemiluminescence signal may include unpolarized, p-polarized and/or s-polarized signals.

The present disclosure relates to a system for measuring chemiluminescence, the system comprising:

a) a metallized surface positioned on a surface substrate;
b) a connector molecule attached to the metallized surface for binding or capture of a desired molecule in a testing sample;
c) a detector molecule having an affinity for the desired molecule, wherein the detector molecule comprises a chemiluminescence label;
d) a triggering component that chemically reacts with the chemiluminescence label to generate a chemically induced electronically exited state; and
e) a measuring device to measure surface plasmon coupled emissions.

The present disclosure relates to an assay kit, wherein the assay kit comprises

a) a substrate surface comprising a metallized surface;
b) a connector component for attachment to the metallized surface having an affinity for a target component to be determined;
c) a detector molecule having an affinity for the target component, wherein the detector molecule comprises a chemiluminescence label;
d) a triggering component that chemically reacts with the chemiluminescence label to generate a chemically induced electronically exited state.

A still further aspect of the present invention relates to the use of low power microwave energy directed at the detection system comprising at least metallic particles for heating of the metallic and/or chemical components therein to enhance the detection system and increase the speed of chemical reactions therein.
The present disclosure relates to a method for increasing and enhancing chemiluminescence signals, the method comprising;

(a) applying a metallic material to a surface or within such surface used in a detection system;
(b) introducing a solution containing at least one biomolecule for disposing near the metallic surface, wherein the biomolecule comprises a chemiluminescent label;
(c) triggering the chemiluminescent label to induce a chemically electronically excited state thereby generating metallic surface plasmons;
(d) irradiating the system with microwave energy; and
(e) measuring the chemiluminescence signal.

Other features and advantages of the invention will be apparent from the following detailed description, drawings and claims.

BRIEF DESCRIPTION OF THE FIGURES

Figure 1 shows Metal-Enhanced Chemiluminescence (MEC) on a silvered surface, Top, and photographs showing the enhanced luminescence, Bottom.

Figure 2 shows the metal-enhanced chemiluminescence on a silvered surface as a function of time, Top, and the intensity of luminescence in terms of seconds, Bottom.

Figure 3 shows the proposed model for Metal-Enhanced Chemiluminescence (MEC). The chemically induced electronically excited luminophore (C) transfers energy to silver plasmons (a resonance coupling interaction), which themselves radiate the photophysical properties of the excited species. CL - Chemiluminescence, MEC - Metal-Enhanced Chemiluminescence, Ag - Silver.

Figure 4 shows the experimental sample set-up wherein the chemiluminescence species is placed between two glass slides comprising silver islands deposited thereon.

Figure 5 shows the chemiluminescence intensity measured on both SiFs and glass as a function of time (Top) and the data normalized (Top-insert). Normalized chemiluminescence intensity on both SiFs and a continuous silver film (Bottom). Photograph of the emission from both the continuous silver film and the SiFs (Bottom - insert). Ag - Silver. SiFs - Silver Island Film.

Figure 6 shows the Experimental geometry used for measuring and/or detecting surface plasmon-coupled chemiluminescence (SPCC). Top, view from the top; bottom, side view.

Figure 7 shows surface plasmon-coupled chemiluminescence from 20-nm-thick aluminum films. Top right, enlarged directional SPCC; top left, free-space chemiluminescence and SPCC; bottom, emission spectra ofboth the free-space chemiluminescence and SPCC.

Figure. 8 shows surface plasmon-coupled chemiluminescence from 45-nm-thick silver films. Top right, enlarged directional SPCC; top left, free-space chemiluminescence and SPCC; bottom, emission spectra of both the free-space chemiluminescence and SPCC.

Figure 9 shows surface plasmon-coupled chemiluminescence from 42-nm-thick gold films. Top right, enlarged directional SPCC; top left, free-space chemiluminescence and SPCC; bottom, emission spectra of both the free-space chemiluminescence and SPCC.

Figure 10 shows photographs of the coupled emission at various polarizations for gold, silver, and aluminum films, top to bottom, respectively, taken at their respective SPCC peak angles. See location of camera in Figures 7-9 (top right).

Figure 11 shows surface plasmon-coupled chemiluminescence (SPCC) and free-space chemiluminescenoe from a small sample chamber, top left, and the enlarged coupled region, top right. Bottom, emission spectra of both free-space chemiluminescence and SPCC from the small chamber.

Figure 12 shows that chemiluminescence intensity decays from aluminum films for both free space and coupled (top) and normalized to the same initial intensity (bottom).

Figure 13 shows that chemiluminescence intensity decays from silver films for both free space and coupled (top) and normalized to the same initial intensity (bottom).

Figure 14 shows a setup for HRP-acridan chemiluminescence assay on both glass and silvered slides.

Figure 15 shows 3D plots of acridan assay emission as a function of time from glass slides without (top) and with low-power microwave exposure/pulses (middle). (Bottom) Photographs showing the acridan emission both before (a) and after a low-power microwave pulse (b). Mw, microwave pulse. The concentration of BSA-biotin was 1.56 pM. 3D plots of the acridan assay chemiluminescence emission as a function of time from silvered glass slides (Ag) without and with low-power microwave exposure/pulses were generated (data not shown). Photographs showing the acridan emission both before (a) and after a low-power microwave pulse (b) were also generated (not shown). Mw, microwave pulse. The concentration of BSA-biotin was 1.56 pM.

Figure 17 shows 3D plots of the acridan assay chemiluminescence emission from both glass (top) and silvered substrates (bottom). (Right) Emission spectra are the average of 400 1-s time points. In both cases, BSA-biotin was *not* immobilized to the surfaces, which were exposed to microwave pulses at 100- and 200-s time points. The final concentration of HRP-streptavidin in the assay was ~10 μg/mL.

Figure 18 shows the acridan chemiluminescence emission intensity as a function of time for different concentrations of surface-bound BSA-biotin. a, 156 pM BSA-biotin; b, 15.6 pM BSA-biotin; c, 1.56 pM BSA-biotin; d, 156 fM BSA-biotin; and e, no BSA-biotin.

Figure 19 shows photon flux integrated over 500 s of the assay shown in Figure 18, for different concentrations of BSA-biotin from both glass and silvered surfaces (Ag). Baselines correspond to integrated photon flux over 500 s for glass and silvered surfaces (Ag) incubated with 1% BSA solution and streptavidin HRP.

Figure 20 shows the procedure for the MT-MEC immunoassay (Mw, low-power microwave heating).

DETAILED DESCRIPTION OF THE INVENTION

Surface plasmons are collective oscillations of free electrons at metallic surfaces. When a metallic article or surface is exposed to an electromagnetic wave, the electrons in the metal (plasmons) oscillate at the same frequency as the incident wave. Subsequently, the oscillating electrons radiate electromagnetic radiation with the same frequency as the oscillating electrons. It is this re-radiation of light at the same incident wavelength that is often referred to as plasmon emission. In the present invention chemically induced electronic excited states (chemiluminescence species) couple to surfaces plasmons to produce emission intensities greater than from about 5 to 1000-fold, as compared to a control sample containing no metallic surface. This approach is of significance for optically amplifying chemiluminescence based clinical assays, potentially increasing analyte/biospecies detectability.

The term "biomolecule" means any molecule occurring in nature or a derivative of such a molecule. The biomolecule can be in active or inactive form. "Active form" means the biomolecule is in a form that can perform a biological function. "Inactive form" means the biomolecule must be processed either naturally or synthetically before the biomolecule can perform a biological function. Exemplary biomolecules include nucleic acids, aromatic carbon ring structures, NADH, FAD, amino acids, carbohydrates, steroids, flavins, proteins, DNA, RNA, oligonucleotides, peptide, nucleic acids, fatty acids, myoglobin, sugar groups such as glucose etc., vitamins, cofactors, purines, pyrimidines, formycin, lipids, phytochrome, phytofluor, peptides, lipids, antibodies and phycobiliproptein.

The term "receptor-ligand" as used herein means any naturally occurring or unnaturally occurring binding couple wherein the components have affinity for each other. For example, the binding couple may include an antibody/antigen complex, viral coat ligand/protein cell receptor or any combination of probe and binding partner. The term "receptor" refers to a chemical group, molecule, biological agent, naturally occurring or synthetic that has an affinity for a specific chemical group, molecule, virus, probe or any biological agent target in a sample. The choice of a receptor-ligand for use in the present invention will be determined by nature of the disease, condition, or infection to be assayed.

Embodiments of the present invention are applicable to chemiluminescence labels or moieties which participate in light-producing reactions in the presence of a triggering agent or cofactor. In the present application, for purposes of example and without.limitation, a preferred embodiment will be discussed in terms of chemiluminescence labels and triggering agent. The label affixed to the detector molecule will be referred to as the "label" or "label agent". For purposes herein, "triggering agent or cofactor" is broadly used to describe any chemical species, other than the chemiluminescence labels which participates in a reaction and which produces a detectable response. Chemiluminescence labels and triggering agents produce a light response.

Examples of suitable chemiluminescenee labels include but without limitation, peroxidase, bacterial luciferase, firefly luciferase, functionalized iron-porphyrin derivatives, luminal, isoluminol, acridinium esters, sulfonamide and others. A recent chemiluminescent label includes xanthine oxidase with hypoxanthine as substrate. The triggering agent contains perborate, an Fe-EDTA, complex and luminol. Choice of the particular chemiluminescence labels depends upon several factors which include the cost of preparing labeled members, the method to be used for covalent coupling to the detector molecule, and the size of the detector molecules and/or chemiluminescence label. Correspondingly, the choice of chemiluminescence triggering agent will depend upon the particular chemiluminescence label being used.

Chemiluminescent reactions have been intensely studied and are well documented in the literature [39]. For example, peroxidase is well suited for attachment to the detector molecule for use as a chemiluminescence. The triggering agent effective for inducing light emission in the first reaction would then comprise hydrogen peroxide and luminol. Other triggering agents which could also be used to induce a light response in the presence of peroxidase include isobutyraldehyde and oxygen.

Procedures for labeling detector molecules, such as antibodies or antigens with peroxidase are known in the art. For example, to prepare peroxidase-labeled antibodies or antigens, peroxidase and antigens or antibodies are each reacted with N-succinimidyl 3-(2-pyridyldithio) proprionate (hereinafter SPDP) separately. SPDP-labeled peroxidase, or SPDP-labeled antigen or antibody is then reacted with dithiothreitol to produce thiol-labeled peroxidase, or thiol-labeled antigen or antibody. The thiol derivative is then allowed to couple with the SPDF-labeled antigen or antibody, or SPDP-labeled peroxidase.

Techniques for attaching antibodies or antigens to solid substrates are also well known in the art. For example, antibodies may be coupled covalently using glutaraldehyde to a silane derivative of borosilicate glass.

Although chemiluminescence detection has been successfully implemented, the sensitivity and specificity of these reactions require further improvements to facilitate early diagnosis of the prevalence of disease. In addition, most protein detection methodologies, most notably western blotting, are still not reliable methods for accurate quantification of low protein concentrations without investing in high-sensitivity detection schemes. Protein detection methodologies are also limited by antigen-antibody recognition steps that are generally kinetically very slow and require long incubation times; e.g., western blots require processing times in excess of 4 h. Thus, both the rapidity and sensitivity of small-molecule assays are still critical issues to be addressed to improve assay detection.

Thus, in one embodiment, the application of low level microwave heating of the sample may be used to speed up any biological/biochemical kinetics within the system. Notably, low level microwaves do not destroy or denature proteins, DNA, or RNA, but instead heat the sample sufficiently to provide for accelerated kinetics such as binding or hybridization. In addition, the microwaves are not scattered by the low density silver metal, which is contrary to most metal objects, such as that recognized by placing a spoon in a microwave oven.

Microwaves (about 0.3 to about 300 GHz) lie between the infrared and radio frequency electromagnetic radiations. It is widely thought that microwaves accelerate chemical and biochemical reactions by the heating effect, where the heating essentially follows the principle of microwave dielectric loss. Polar molecules absorb microwave radiation through dipole rotations and hence are heated, where as non-polar molecules do not absorb due to lower dielectric constants are thus not heated. The polar molecules align themselves with the external applied field. In the conventional microwave oven cavity employed in this work, the radiation frequency (2450 MHz) changes sign $2.45 \times 10^9$ times per second. Heating occurs due to the tortional effect as the polar molecules rotate back and forth, continually realigning with the changing field, the molecular rotations being slower than the changing electric field. The dielectric constant, the ability of a molecule to be polarized by an electric field, indicates the capacity of the medium to be microwave heated. Thus, solvents such as water, methanol and dimethyl formamide are easily heated, where as microwaves are effectively transparent to hexane, toluene and diethylether.

For metals, the attenuation of microwave radiation arises from the creation of currents resulting from charge carriers being displaced by the electric field. These: conductance electrons are extremely mobile and unlike water molecules can be completely polarized in 10-18 s. In microwave cavity used in the present invention, the time required for the applied electric field to be reversed is far longer than this, in fact many orders of magnitude. If the metal particles are large, or form continuous strips, then large potential differences can result, which can produce dramatic discharges if they are large enough to break down the electric resistance of the medium separating the large metal particles.

Interestingly, and most appropriate for the new assay platform described herein, small metal particles do not generate sufficiently large potential differences for this "arcing" phenomenon to occur. However, as discuss hereinbelow, the charge carriers which are displaced by the electric field are subject to resistance in the medium in which they travel due to collisions with the lattice phonons. This leads to Ohmic heating of the metal nanoparticles in addition to the heating of any surface polar molecules. Intuitively, this leads to localized heating around the metallic nanostructures in addition to the solvent, thereby rapidly accelerating assay kinetics.

In the present invention, microwave radiation may be provided by an electromagnetic source having a power level in a range between about 100 watts to 150 watts. Any source, known to one skilled in the art may be used, such as a laser having the capacity to emit energy in the microwave range. The microwave radiation may be emitted continuously or intermittently, as desired, to maintain the metallic particles at a predetermined temperature such that it is capable of increasing the speed of chemical reactions not only in the assay system but also the chemiluminescence species.

In the alternative, microwave energy can be supplied through a hollow wave guide for conveying microwave energy from a suitable magnetron. The microwave energy is preferably adjusted to cause an increase of heat within the metallic material without causing damage to any biological materials in the assay system.

In one embodiment the present invention provides for a metallic surface and a biomolecule capable of chemiluminescing, wherein the metallic surface and the biomolecule are separated by at least one film spacer layer. The thickness of said film may be chosen so as to enhance the chemiluminescence of the biomolecule by positioning the biomolecule an optimal distance from the metallic surface. The film spacer layer may be one or multiple layers of a polymer film, a layer formed from a fatty acid or a layer formed from an oxide. In a preferable embodiment, the film spacer layers and the metallic surface are chemically inert and do not bind to the biomolecules to be detected or to intermediates that are bound to the compounds to be detected, for example covalently bound. The layer formed from a fatty acid may be formed by a Langmuir-Blodgett technique. The film spacer layer may be a spin coated polymer film. The oxide layer may be formed from a deposition technique, such as vapor deposition.

Further, the metallic surface may be in the form of a porous three dimensional matrix. The three dimensional matrix may be a nano-porous three dimensional matrix. The metallic surface may include metal colloid particles and/or metal-silica composite particles. The metallic surface may comprise agglomerated metal particles and/or binary linked particles or metal particles in a polymer matrix. The three dimensional matrix may be formed from controlled pore glasses or using matrices assembled from the aggregation of silver-silica composites themselves. The matrices may be metallic nanoporous matrix, through which species will flow and be both detected and counted more efficiently. The ability to quantitatively count single flowing molecules under practical conditions may have many implications for medical diagnostics, the detection of biohazard organisms and new and quicker methods for DNA sequencing.

The emission enhancement may be observed at distances according to the type of chemiluminescence species to be detected and the type of metal. For example, emission enhancement may be observed when a chemiluminescence species is positioned about 5 nm to about 200 nm to metal surfaces. Preferable distances are about 5 nm to about 30 nm, and more preferably, 5 nm to about 20 nm to metal surfaces. At this scale, there are few phenomena that provide opportunities for new levels of sensing, manipulation, and control, In addition, devices at this scale may lead to dramatically enhanced performance, sensitivity, and reliability with dramatically decreased size, weight, and therefore cost.

Different effects are expected for mirrors, sub-wavelength or semi-transparent metal surfaces, silver island films or metal colloids. More dramatic effects are typically observed for islands and colloids as compared to continuous metallic surfaces. The silver islands have the remarkable effect of increasing the emission intensity at least 5-fold while decreasing the lifetime 100-fold.

Light from the chemiluminescence reaction generated by the random depopulation of a chemically induced electronic state of a luminophore and/or the plasmons coupled emissions from the metallic components can be detected using an optical detector, positioned above and/or below reaction sites. Various optical detectors, such as photodiode, charge-coupled device (CCD), photomultiplier tube (PMT), or photon counting detector, have different degree of sensitivity. PMT and photon counting detectors can achieve an electronic amplification factor as high as $10^6$-$10^8$. Conventional PMTs require a ~1 kV power source, but new miniaturized detector requires: only a 5 V. Most of the chemiluminescence emission wavelengths are in the visible region. A narrow-band optical filter may be used to ensure detecting luminescence wavelengths. The system may include a microactuator, detector, microprocessor, electronics, a display, and translation stage. The output of the detector may be interfaced to an analog to digital converter and a microprocessor to calculate analyte concentration.

It is known that the extinction properties ($C_E$) of metal particles can be expressed as both a combination of both absorption ($C_A$) and scattering ($C_S$) factors, when the particles are spherical and have sizes comparable to the incident wavelength of light, i.e. in the Mie limit[26].

$$C_E = C_A + C_S = k_1 \operatorname{Im}(\alpha) + \frac{k_1^4}{6\pi}|\alpha|^2 \quad (1)$$

where $k_1 = 2\pi n_1 / \lambda_0$ is the wavevector of the incident light in medium I and $\alpha$ is the polarizability of a sphere with radius r, $n_1$ is the refractive index and $\lambda_0$ the incident wavelength. The term $|\alpha|^2$ is square of the modulus of $\alpha$.

$$\alpha = 4\pi r^3 (\varepsilon_m - \varepsilon_1)/(\varepsilon_m + 2\varepsilon_1) \quad (2)$$

where $\varepsilon_1$ and $\varepsilon_m$ are the dielectric and the complex dielectric constants of the metal respectively. The first term in equation 1 represents the cross section due to absorption, $C_A$, and the second term, the cross section due to scattering, $C_S$. Current interpretation of metal-enhanced fluorescence [23] is one underpinned by the scattering component of the metal extinction, i.e. the ability of fluorophore-coupled plasmons to radiate (plasmon scatter) [11]. Intuitively, larger particles have wavelength distinctive scattering spectra ($C_S$) as compared to their absorption spectra ($C_A$) [26], facilitating plasmon coupled emission from the larger nanoparticles.

Surprisingly, the present invention shows that chemically induced electronic excited states (chemiluminescence species) also couple to surface plasmons, producing emission intensities from about 5 to about 1000 fold, as compared to a control sample containing no surface silver nanostructures. Thus, the present invention further shows that surface plasmons can be directly excited by chemically induced electronically excited luminophores.

The present invention provides enhanced emissions using metallized nanostructures, islands of elliptical, spherical, triangular or rod-like forms. In exemplary cases, the elliptical islands have aspect ratios of 3/2, and the spherical colloids have diameters of 20-60 nm. However, the invention is not limited to any particular geometry. Using known coating techniques, the placement of metallic islands could be controlled precisely, as close as 50 nm apart.

Metal island particles may be prepared in clean beakers by reduction of metal ions using various reducing agents [10-13 and 27]. For example, sodium hydroxide is added to a rapidly stirred silver nitrate solution forming a. brown precipitate. Ammonium hydroxide is added to re-dissolve the precipitate. The solution is cooled and dried quartz slides are added to the beaker, followed by glucose. After stirring for 2 minutes, the mixture is warmed to 30°C. After 10-15 minutes, the mixture turns yellow-green and becomes cloudy. A thin film of silver particles has formed on the slides as can be seen from their brown green color. The slides are rinsed with pure water prior to use.

Alternative procedures for preparing metal particles are also available [28-32]. Silver is primarily used because of the familiar color from the longer surface plasmon absorption of silver.

Colloids can be prepared as suspensions by citrate reduction metals. Preferred metals are silver and gold. The size of the colloids and their homogeneity can be determined by the extensive publications on the optical properties of metal particles available and the effects of interface chemistry on the optical property of colloids [33].

Silver island films can be formed by a chemical reduction of a silver salt on the quartz surface and that are relatively simple to fabricate. However, this approach does not provide a control of particle size, or distance of the chemiluminescent species from the metallic surface.

Metal particles can be bound to a surface by placing functional chemical groups such as cyanide (CN), amine ($NH_2$) or thiol (SH), on a glass or polymer substrate. Metal colloids are known to spontaneously bind to such surfaces with high affinity [34-35].

Positioning of the biomolecule or metal particle at a desired distance can be achieved by using a film. The film may be a polymer film, a Langmuir-Blodgett film or an oxide film. Proper distances may be achieved by using Langmuir-Blodgett films with fatty acid spacers. The fatty acids may be from natural sources, including concentrated cuts or fractionations, or synthetic alkyl carboxylic acids. Examples of the fatty acids include, but not limited to, caprylic ($C_8$), capric ($C_{10}$), lauric ($C_{12}$), myristic ($C_{14}$), palmitic ($C_{16}$), stearic ($C_{18}$), oleic ($C_{18}$), linoleic ($C_{18}$), linolenic ($C_{18}$), ricinoleic ($C_{18}$) arachidic ($C_{20}$), gadolic ($C_{20}$), behenic (C22) and erucic ($C_{22}$). The fatty acids with even numbered carbon chain lengths are given as illustrative though the odd numbered fatty acids can also be used.

Also, metal-chemiluminescence species distances may be achieved by using polymer films. Examples of the polymer include, but not limited to, polyvinyl alcohol (PVA). Absorbance measurements and ellipsometry may be used to determine polymer film thickness. One type of polymer films is spin coated polymer films. The technology of spin coated polymer spacer films readily allows films to be coated onto a variety of surfaces, with varied thickness from >0.1 *u*m. The coating can be performed on a spin coater, which allows uniform surface thickness by varying polymer concentration (viscosity) and spin speed. For example, Model P6700 spin coater (Specialty Coating Systems Inc.) allows uniform surface thickness by varying polymer concentration (viscosity) and spin speed.

Metallic colloids (or various other non-spherical shapes/particles) may also be incorporated into organic polymers, covalently or non-covalently, to form polymeric matrices, wherein the distance from diffusing species affords an increase in radiative decay rate and thus, an increase in quantum yield. Such polymeric matrices are ideal for sensing/flowing sensing applications of low concentration species.

Any chemiluminescent species may be used in the present invention that provides for a chemical reaction which produces the excited state responsible for the observed emission including, but not limited to the following excitation mechanisms:

$$R\bullet + R'\bullet \rightarrow R\text{-}R + h\nu$$

(single bond formation (radical-radical reaction))

$$\bullet R\bullet + \bullet R\bullet' \rightarrow R{=}R + h\nu$$

(double bond formation (radical-radical reaction)) $RO_2$ $\bullet R\bullet + O_2 \xrightarrow{\Delta} R + h\nu$

$$R^+ + e^- \rightarrow R + h\nu$$

(electron capture)

This embodiment of the present invention may have vast applications in clinical medicine, environmental monitoring applications, homeland security such as rapid detection of low concentration species, industrial processes, pharmaceutical industries such as monitoring species, and sensors for use in reduced atmospheres such as biohazard clean rooms and environments using space light.

## Examples

### 1. Radiating Plasmons Generated from Chemically Induced Electronic Excited States

### 1.2 Materials

Silver nitrate (99.9%), sodium hydroxide (99.996%), ammonium hydroxide (30%), trisodium citrate, *D*-glucose and premium quality APS-coated glass slides (75×25 mm) were obtained from Sigma-Aldrich (St. Loius, MO). The blue-glow chemiluminescence sticks used were the "Color Bright" light sticks, obtained from Omniglow (West Springfield, MA).

### 1.3 Chemiluminescence

The chemiluminescent materials used in this study were obtained from commercial light glow sticks. These glow sticks contain the necessary reacting chemicals encapsulated within a plastic tube. The plastic tube contains a phenyl oxalate ester and a fluorescent probe, where the choice of dye simply determines the color of the luminescence [9]. For the examples set forth herein, this choice is arbitrary as long as the luminophore emits in the visible spectral region, consistent with previous reports [10-13]. Inside the plastic tube lies a glass capsule containing the activating agent (hydrogen peroxide). Activation of the chemicals is accomplished with a bend, snap, and a vigorous shake of the plastic tube which breaks the glass capsule containing the peroxide and mixes the chemicals to begin the chemiluminescence reaction. The hydrogen peroxide oxidizes the phenyl oxalate ester to a peroxyacid ester and phenol. The unstable peroxyacid ester decomposes to a peroxy compound and phenol, the process chemically inducing an electronic excited state.

1.4 Formation of Silver Island Films (SiFs) on APS-coated Glass Substrates

The silver island films were made according to previously published procedures employing the chemical reduction of silver nitrate on glass microscope slides using sodium hydroxide, ammonium hydroxide and glucose [10-13].

1.5 Chemiluminescence from SiFs and glass

The chemiluminescence experiments were performed using a blue emission glow stick. After chemiluminescence initiation, approximately 70 μl of the glow stick fluid was placed between two APS-coated microscope glass slides, clamped together. The glass slides contained silver island films on one end and were bare glass on the other end. The bare end of the glass served as the control sample by which to compare the benefits of using the metal-enhanced chemiluminescence phenomenon. Subsequently, the enhancement ratio, the intensity from silver / intensity from glass, could be determined.

1.6 Chemiluminescence measurements

Chemiluminescence spectra were collected using an Ocean Optics spectrometer, model SD 2000 (Dunedin, FL), connected to an Ocean Optics 1000 μm diameter fiber with an *NA* of 0.22 (Dunedin, FL). The fiber was positioned vertically on top of the slides containing the luminescening material. Spectra were collected with an integration time ranging from between 4 and 10 seconds. The integration time was kept constant between the control and silver island film sample measurements.

1.7 Results

Figure 1 top shows the luminescence emission spectra from between the silvered glass and glass plates. The emission from the silvered portion of the slide was spatially averaged to be about 4-5 times greater than the glass control side of the sample. In addition, the volume between both the sandwiched glass and silver slides was identical. Figure 1 - bottom shows the photographs of the slides, both before and after the addition of the chemiluminescent material. Approximately 70 μL of fluid was enough to form a thin coating across both portions of the slide, held by capillary action as the slides were sandwiched as shown in Figure 4. The enhanced chemiluminescence is clearly visible on the silvered portion as shown in Figure 1 (bottom). Interestingly, the digital camera was not able to capture the blue emission from the thin fluid layer of the glass region of the slide, the intensity quite weak as also shown in Figure 1-top.

Several control experiments were performed to determine the loss of chemiluminescent intensity, due to the depletion of the reactants, Figure 2. After a period of 60 minutes, most of the emission from the silvered plates had gone, Figure 2- Top. Interestingly, the luminescence emission intensity changed very little in several tens of seconds, Figure 2 - bottom, which was the time needed to measure both the intensity on silver and glass shown in Figure 1, making the comparison between both silver and glass a valid one. Finally, while not shown here, the *rate of loss* of luminescence was measured from both the silvered and glass portions of the slide. For both, the rate of chemiluminescence was almost identical, suggesting that no chemical interaction between the chemiluminescent reagents and silver occurred, the enhanced luminescence signals observed due to interactions with surface plasmons as discussed below [23].

Several detailed control experiments were undertaken to ascertain whether silver could catalyze the chemiluminescence reaction and account for the enhanced optical signatures observed, as compared to an interpretation in therms of a chemiluminescence-based radiating plasmon model. Figure 5 - top shows the luminescence intensity as a function of time. Clearly the enhanced luminescence from the SiFs is visible, with the initial intensity on silver ≈ 3100 a.u. (at t = 0) as compared to < 150 on glass. Subsequently the rates of loss of luminescence were compared after the curves were normalized, Figure 5 - top insert. The rate of loss of luminescence, which is due to the depletion of solution reactants and therefore depletion over time of excited states, was found to follow first order decay kinetics and could simply be modeled to an exponential function of the form:

$$\text{Luminescence Intensity, } I = C + B^{-kt} \quad (3)$$

where C is the intensity at time $t = \infty$, B is a pre-exponential factor and k then rate of luminescence depletion, units $S^{-1}$. From Figure 5 - Top insert, the rate of depletion on silver was found to be 1.7 times faster than on glass, 0.034 vs 0.019 $s^{-1}$ respectively. Two explanations could initially describe this observation: Firstly, silver catalysis of the chemiluminescence reaction, or secondly, the high rate of transfer / coupling of the chemiluminescence to surface plasmons, rapidly reducing the excited state lifetime of the chemiluminescence species.

To eliminate silver based catalysis of the chemiluminescence reaction as an explanation for the enhanced signals, the luminescence rates were measured on both SiFs and a continuous silver strip. Interestingly, the rate of loss of luminescence was still found to be greater on the SiFs as compared to the continuous silver strip, Figure 5 - bottom. In addition, the emission intensity was very low indeed from the continuous strip of silver, Figure 5 - bottom insert. Given that the continuous strip is indeed darker and that the rate is slower than on SiFs, then silver based catalysis can be eliminated as a possible explanation of the observation of increased signal intensities on the SiFs. Subsequently, these observations suggest that chemically induced electronic excited states (chemiluminescence species) can readily induce/couple to surface plasmons, facilitating metal-enhanced chemiluminescence.

1.8 Discussion

With the chemiluminescence species shown here, it is theorized that excited chemiluminescence species couple to surface plasmons, which is turn radiate the photophysical properties of the chemically excited state, as shown in Figure 3. Interestingly, the chemiluminescent system described herein, wherein there is no external excitation source for direct illumination and no direct mode of excitation of the surface plasmons suggests that the surface plasmons are indeed excited from a chemically induced electronically excited state of a luminophore. It is believed that this is the first observation of the chemically induced electronic excitation of surface plasmons.

2. Directional and Polarized Emission of the Luminescence

The experimental geometry used for the surface plasmon-coupled chemiluminescence (SPCC) studies is shown in Figure 6.

2.1 Materials and Methods

Premium quality APS-coated glass slides (75 × 25 mm), silver wire (99.99+% purity), aluminum evaporation slugs (99.999% purity), and silicon monoxide pieces (99.99% purity) were obtained from Sigma-Aldrich (St. Loius, MO). Gold evaporation slugs (99.999% purity) were obtained from Research and PVD Material Corporation (Wayne, NJ). CoverWell imaging chamber gaskets with adhesive (20-mm diameter, 1-mm deep) were obtained from Molecular Probes (Eugene, OR). The smaller imaging chambers were built in-house using electrical black tape, double sticky tape, and microscope coverslips. Several standard chemiluminescence kits from Omnioglow (West Springfield, MA) and Night Magic (Union City, OH) were used as the source of chemiluminescence.

2.2 Chemiluminescent Dyes

The chemiluminescent materials used in this study were obtained from commercially available kits and previously described in Example 1.

2.3 Formation of Continuous Thin Films of Metal on APS-Coated Glass Substrates

Twenty nanometers of aluminum, 45 nm of silver, and 40 nm of gold were deposited on separate APS-coated glass slides using an Edwards Auto 306 Vacuum Evaporation chamber (West Sussex, U.K.) under ultrahigh vacuum ($<3 \times 10^{-6}$ Torr). In each case, the metal deposition step was followed by the deposition of 5 nm of silica via evaporation without breaking vacuum. This step served to protect the metal surface from attack by the various chemical species present in the chemiluminescence assay.

2.4 Surface Plasmon-Coupled Chemiluminescence (SPCC) of Dyes on Continuous Metal Films

The surface plasmon-coupled chemiluminescence (SPCC) experiments were performed using several different colors of the chemiluminescent dyes ranging from blue to red. They were carried out by first bending the plastic tube of the chemiluminescence kit and shaking it vigorously. This allowed the reaction mixtures to mix and begin to luminesce. The tubes were then cut with a scissor, and the reacting fluid was poured into a glass vial. Approximately 150 μL of the reacting fluid was then placed in an imaging chamber gasket with adhesive (20-mm diameter, 1-mm deep). This gasket was then pressed against an (APS-coated) continuous metal-coated and silica-capped microscope glass slide until they were stuck together creating a chamber containing the chemiluminescent dyes on the surface of the metal-coated glass slide. For smaller samples, approximately 50 μL of the reacting fluid was placed in an imaging chamber built in-house attached to an (APS-coated) continuous metal-coated and silica-capped microscope glass slide.

2.5 Surface Plasmon-Coupled Chemiluminescence (SPCC) Measurements

The metal-coated slides containing the chemiluminescent dyes were attached to a hemicylindrical prism made with BK7 glass (*n* = 1.52), and the refractive index was matched using spectrophotometric grade glycerol (*n* = 1.475) between the back of the glass slide (uncoated side) and the prism. This unit was then placed on a precise 360° rotatory stage which was built in-house. The rotatory stage allowed the collection of light at all angles around the sample chamber. An Ocean Optics low OH 1000 μm diameter optical fiber with NA of 0.22 (Dunedin, FL) used for collecting the chemiluminescence signals was mounted on a holder that was screwed onto the base of the rotatory stage. A pictorial representation of the top and side view of the setup is presented in Figure 6. Surface plasmon-coupled chemiluminescence (SPCC) spectra were collected using a model SD 2000 Ocean Optics spectrometer (Dunedin, FL) connected to the above-mentioned optical fiber. The spectra were collected with an integration time between 0.5 and 2 s (depending on the intensity of the various SPCC signals). Both unpolarized and p- and s-polarized signal information was collected for the SPCC signal (from 0 to 180° with respect to the front of the prism) and for the free-space signal (from 180 to 360° with respect to the front of the prism). A separate time-dependent decay study was performed on each chemiluminescent dye to study the comparative time-dependent decay profile of the SPCC signal and the free-space signal.

2.6 Results

Figure 7 (top left) shows the surface plasmon-coupled chemiluminescence (SPCC) and the free-space emission from the blue chemiluminescent dye on a 20-nm aluminum layer. It can be seen that the free-space emission is of much higher magnitude than the SPCC signal. This is because the sample chamber is 1-mm thick and only the luminophores within approximately 250 nm of the surface of silver are known to excite surface plasmons [36, 24]. Hence, the majority of the luminophores in the chamber do not couple to plasmons and so radiate their energy in the form of free-space emission. Subsequently there was an attempt to use very thin films of liquid to alleviate this effect. However, the hydrophobic nature of the surface globulated the chemiluminescence liquid, preventing films <250 nm thick to be produced.

Figures 7 (top right) is an enlarged figure showing the highly directional and predominantly p-polarized SPCC emission only, suggesting that the observed signal is due to surface plasmons. This is in stark contrast to the free-space emission which does not show any polarization or directional preference. However, the signal at the SPCC peak angle is not entirely p-polarized. The camera located at the SPCC peak angle of the figure depicts the approximate angular position where photographs of the coupled emission at various polarizations were taken. These photographs are shown in Figure 10. Figure 7 (bottom) is the normalized SPCC and free-space emission spectra showing a high degree of overlap between the spectra. This suggests the plasmon-coupled chemiluminescence has not undergone any changes in its spectral properties because of the interaction between the luminescent species and the metal surface.

Figure 8 (top left) shows the surface plasmon-coupled chemiluminescence (SPCC) and the free-space emission from the green chemilummescent dye on a 45-nm silver layer. Similar to the case of the blue dye on aluminum, it can also be seen here that the free-space emission is of greater magnitude than the SPCC signal. Figure 8 (top right) is an enlarged figure showing the highly directional and predominantly p-polarized SPCC emission only, suggesting that the observed signal is due to surface plasmons. This again is in stark contrast to the free-space emission which does not show any polarization or directional preference. Figure 8 (bottom) is the normalized SPCC and free-space emission spectra showing a high degree of overlap between the spectra, suggesting no additional interaction between the luminescent species and the metal surface.

Figure 9 (top left) shows the surface plasmon-coupled chemiluminescence (SPCC) and the free-space emission from the red chemiluminescent dye on a 42-nm gold layer. Figure 9 (top right) is an enlarged figure showing the highly directional and predominantly p-polarized SPCC emission only, suggesting that the observed signal is due to surface plasmons. The SPCC again is in stark contrast to the free-space emission which does not show any polarization or directional preference. Figure 9 (bottom) is the normalized SPCC and free-space emission spectra showing a high degree of overlap between the spectra, suggesting no other interaction between the luminescent species and the metal surface.

Figure 10 shows photographs of the coupled emission (from the prism side) at the respective SPCC peak angle from the various dyes at both s- and p-polarizations as well as with no polarization. The approximate angular location of the camera used obtaining these photographs is marked in Figures 7-9 (top right). This figure clearly shows that the emission at the SPCC peak angle is predominantly p-polarized for all three dyes (on all three metals) thus suggesting that surface plasmons are responsible for the SPCC signal. It can be seen that the p-polarized signal intensity at the SPCC peak angle is lower in magnitude than the unpolarized signal. This occurs because the entire SPCC signal consists of both p- and to a lesser degree s-polarized light, and also because the sheet polarizers used in the experiment have only 30-40% peak transmission efficiency for both polarizations.

Initially, the broadness of the SPCC peak angles for all three dyes which varied between 20 and 25 degrees. Hence, to investigate whether the broadness of the SPCC peak angle is a function of the surface area of the sample, the experiments were repeated on silver using the green chemiluminescent dye with a sample chamber that had approximately half the surface area when compared to the samples made with commercially available imaging chambers that had been used thus far. Figure 11 (top left) shows the surface plasmon-coupled chemiluminescence (SPCC) and the free-space emission from the green chemiluminescent dye on a 45-nm silver layer for the small imaging chambers. Figure 11 (top right) is an enlarged figure showing the highly directional and predominantly p-polarized SPCC emission only. Here, the broadness of the SPCC peak angle is approximately 20 degrees. It is clear from this figure that the broadness of the SPCC peak angle is not significantly affected by the surface area of the sample. An interesting observation in Figure 11 (top right) is the decay in the SPCC signal in the region between 90 and 180 degrees when compared to that in the 0-90 degrees. This is because the data was collected sequentially from 0 through 360 degrees. As a result, for the small chamber with a lower volume of reactants, by the time the data in the region between 90 and 180 degrees was collected, a signal reduction is observed because of the depletion of reactants (depletion of excited states) over time. The broad angle distribution shown in Figures 7-9 and 11 is attributed to the waveguide effect, given that our solution of chemiluminescence occupied a sample chamber of 1-mm thickness. Figure 11 (bottom) is the normalized SPCC and free-space emission spectra showing a high degree of overlap between the spectra, suggesting no additional interaction between the luminescent species and the metal surface in the smaller imaging chambers built in-house.

The next round of experiments was performed to determine the rate of decay of luminescence for the blue and green chemiluminescent dyes as a function of time for both the free-space emission and the SPCC emission (with p-polarizers so that only plasmon-coupled emission was measured). By decay rate, it is meant the decrease in intensity because of depletion of reagents. The results of these experiments for the blue dye on aluminum and green dye on silver are shown in Figures 12 and 13, respectively. Figure 12 (top) shows the decay of free-space and SPCC emission as a function of time for the blue dye on aluminum, with Figure 12 (bottom) image showing both the decay intensities normalized to their respective values at t = 0. The rate of loss of luminescence, which is due to the depletion of solution reactants and therefore a depletion over time of excited states, was found to follow first-order decay kinetics as shown herein above in formula (3).

The rate of depletion of the SPCC signal for the blue dye on aluminum was found to be only minimally greater than the free-space emission, 0.0003 versus 0.0002 $s^{-1}$, respectively. Since both the SPCC signal and the free-space emission signal decay are highly dependent on the rate of depletion of the same reactants (depletion of excited states) in the sample chamber over time, it is not surprising that the measured decay rates for both the signals as shown in Figure 12 are almost identical. However, this finding does indicate that there are no localized catalytic effects of the aluminum on the chemiluminescence reaction, as this would be expected to manifest in a larger difference in the SPCC luminescence decay rate (from the free-space decay rate) than is currently observed.

Figure 13. (top) shows the decay of free-space and SPCC emission as a function of time for the green chemiluminescent dye on silver, and Figure 13 (bottom) shows both the decay intensities normalized to their respective values at $t$ = 0. The rate of depletion of the SPCC signal for the green dye on silver was found to be only minimally smaller than the free-space emission, 0.0005 versus 0.0006 $s^{-1}$, respectively. It is again not surprising that the measured decay rates for both the signals as shown in Figure 12 are almost identical, since both the SPCC signal and the free-space emission signal decay are highly dependent on the rate of depletion of the same reactants in the sample chamber over time. This finding again indicates no localized catalytic or chemical effects of the silver on the chemiluminescence reaction studied.

2.7 Conclusions

The results of this study lead us to conclude that chemically induced electronic excited states of luminophores can excite surface plasmons on thin films of continuous metal, producing highly polarized and directional emission. This phenomenon is not restricted to the commercially available kits that were used in this study but rather can be extended to the myriad of chemiluminescent reactions employed in biotechnology today to increase signal collection efficiency and hence the sensitivity of such assays. The typical thickness of the functional surface of such assays are compatible with an approximately 250-nm coupling region, potentially alleviating unwanted background signals caused by spontaneous reaction of reagents or unwanted enzymatic activity and therefore increasing assay sensitivity.

Another interesting observation is that SPCC occurs with gold films. Since luminophores within approximately 250 nm of the surface of metal are known to excite surface plasmons, which is longer than the distances required for nonradiative quenching of luminescence, the potential of using gold as the metal surface becomes an advantage. This is because gold is chemically more stable than silver and the surface chemistry of gold is well-known and characterized [37]. Also, since gold films are widely used in surface plasmon resonance (SPR), this provides a robust technology base for the mass production of suitable gold films.

3. Microwave Triggered Metal Enhanced Chemiluminescence

3.1 Materials

Bovine-biotinamidocaproyl-labeled albumin (biotinlyated BSA), HRP-labeled avidin, silver nitrate (99.9%), sodium hydroxide (99.996%), ammonium hydroxide (30%), trisodium citrate, D-glucose, and premium quality APS-coated glass slides (75 × 25 mm) were obtained from Sigma-Aldrich. CoverWell imaging chamber gaskets with adhesive (20-mm diameter, 1 mm deep) were obtained from Molecular Probes (Eugene, OR). Steptavidin-HRP prediluted solution was obtained from Chemicon International Inc. Chemiluminescence materials were purchased from Amersham Biosciences, (ECL Plus Western blotting detection kit, RPN2132). ECL Plus utilizes a new technology, developed by Lumigen Inc., based on the enzymatic generation of an acridinium ester, which produces intense light emission at ~430 nm.

3.2 Formation of Silver Island Films on APS-Coated Glass Substrates

In a typical SiF preparation, a solution of silver nitrate (0.5 g in 60 mL of deionized water) in a clean 100-mL glass beaker, equipped with a Teflon-coated stir bar, is prepared and placed on a Corning stirring/hot plate, While stirring at the quickest speed, 8 drops (~200 $\mu$L) of freshly prepared 5% (w/v) sodium hydroxide solution are added. T his results in the formation of dark brown precipitates of silver particles. Approximately 2 mL of ammonium hydroxide is then added, drop by drop, to redissolve the precipitates. The clear solution is cooled to 5° C by placing the beaker in an ice bath, followed by soaking the APS-coated glass slides in the solution. While keeping the slides at 5 C, a fresh solution of D-glucose (0.72 g in 15 mL of water) is added. Subsequently, the temperature of the mixture is then warmed to 30°C. s the color of the mixture turns from yellow-green to yellow-brown, and the color of the slides become green, the slides are removed from the mixture, washed with water, and sonicated for 1 min at room temperature. SiF-deposited slides were then rinsed with deionized water several times and dried under a stream of nitrogen gas. Prior to assay fabrication and subsequent chemiluminescent experiments, imaging chamber gaskets with adhesive (20-mm diameter, 1 mm deep) were pressed against the silver-coated and silica-capped microscope glass slides until they were stuck together, creating a chamber.

3.3 Preparation of the Model Protein Assay (Biotin-Avidin) on Silver Island Films and on Glass

The model assay used in the present experiment is based on the well-known interactions of biotin and avidin. Biotin groups are introduced to the glass and silvered surfaces through biotinylated BSA, which readily forms a monolayer on the surfaces of glass and SiFs. Biotinylated BSA is bound to SiFs and the glass by incubating 20 $\mu$L of biotinylated BSA solutions with different concentrations in the imaging for ~1 h. Chambers were washed with water to remove the unbound material. Imaging chambers were then incubated with 20 $\mu$L of 1% aqueous BSA (w/v) for 1 h to minimize nonspecific binding of HRP-streptavidin to surfaces. Chambers were again washed with water to remove the BSA blocking solution. Stock solutions of HRP-streptavidin were diluted 1:10 to a final concentration of 100 $\mu$g/mL. Twenty microliters of the HRP-streptavidin solution was subsequently added into the biotinylated BSA-coated glass and SiF-coated imaging chambers and typically microwaves for 20 s in the microwave cavity (0.7 $ft^3$, GE compact microwave model JES735BF, max power 700 W). The power setting was set to 2, which corresponded to 140 W over the entire cavity. In all the experiments performed with low-power microwaves, there was no evidence of surface drying. Following incubation, imaging chambers were again washed with water to remove unbound HRP-streptavidin material prior to the chemiluminescence experiments.

3.4 Chemiluminescence Reagents

The ECL Western blotting detection kit contained two reagents that yield a bright chemiluminescent emission at 430 nm upon mixing. Solution A contained the substrate solution (peroxide formulation), and solution B contained the solution of the luminescent compound, acridan in dioxane and ethanol. HRP and hydrogen peroxide solution (solution A) catalyze the oxidation of the acridan substrate (solution B). As a result, acridinium ester intermediates are formed and further react with peroxide to generate light emission with a maximum wavelength centered around 430 nm.

3.5 Chemiluminescence from Reagents on SiFs and Glass Surfaces

The chemiluminescence experiments were performed with and without microwave heating inside the microwave cavity. During microwave heating, 30-s pulses were applied at three 100-s intervals. The pulses were applied at 30% power, which corresponded to 210 W over the entire cavity. In order to obtain the same initial chemiluminescence emission for all measurements, all chemiluminescent assays were undertaken by combining 40μL of solution A with 2.0μL of solution B and immediately adding the entire solution to the imaging chamber.

Data collection commenced immediately following addition of reagents and terminated when the photon count returned to baseline. Since the rate of photon emission is directly proportional to enzyme concentration, the photon flux was summed for a fixed time interval to determine the relationship between protein concentration and signal intensity, cf. Figures 19 and 20.

3.6 Chemiluminescence Detection

Chemiluminescence spectra were collected using an Ocean Optics spectrometer, model SD 2000 (Dunedin, FL), connected to an Ocean Optics 1000-mm-diameter fiber with an NA of 0.22. The fiber was positioned vertically on top of the slides containing the chemiluminescent reagents inside the microwave cavity. Chemiluminescent spectra and time-dependent emission intensities were collected with an integration time of 1000 ms for -500 s unless otherwise noted. The integration time was kept constant between the control and silver island film sample measurements. The *real-color* photographs were taken with an Olympus Digital camera (C-740, 3.2 Mega Pixel, 10× Optical Zoom) without the need for optical filters.

3.7 Results

To demonstrate protein detection with microwave triggered metal enhanced chemiluminescence (MT-MEC) on silver island films (SiFs), commercially available chemiluminescent reagents (acridan and peroxide) from Amersham Biosciences was used. The model protein assay was constructed with biotinylated BSA surface-modified substrates (SiFs or glass), horseradish peroxidase-streptavidin (HRP-avidin) and chemiluminescent reagents, as demonstrated in Figure 14.

Biotinylated BSA was incubated on silvered or glass substrates fort ~1 h. A 1% aqueous BSA solution was subsequently added to minimize nonspecific binding of HRP-streptavidin to the surfaces. HRP-streptavidin was then added to the surfaces with bound biotinylated BSA. The strong binding affinity of streptavidin for biotin served as the basis for the quantitative determination of the BSA-biotin species on the glass and silvered surfaces. As a result, chemiluminescent reaction rates for these experiments are proportional to the quantity of bound biotinylated BSA HRP-streptavidin complexes, [38] where the dynamic range of protein concentration is proportional to the total luminescent photon flux for a defined time interval.

Following surface modification of glass and silver surfaces, a comparison was made between traditional chemiluminescence reaction yields with microwave (Mw) "trigger" reaction yields. With the addition of the chemiluminescent mixtures to the functionalized surfaces, the emission data was collected for the MT-MEC assays within the microwave cavity using a fiber optic that is connected to a spectrofluorometer and a computer (not shown). The microwave cavity power was ~140 W. Detection was accomplished through a fiber delivered through a small opening on the top of the microwave cavity. Imaging chambers were placed in the microwave, and wells of interest were aligned with the tip of the fiber to optimize collection efficiencies.

Figure 15 top shows the first 500 s of collection time for the chemiluminescence emission from the glass surfaces. Figure 15, bottom, shows the chemiluminescence emission from the glass substrate under the same initial conditions, but the sample is subjected to 30-s microwave pulses at ~100-s intervals. These results clearly show the "on-demand" nature of microwave-triggered chemiluminescence reactions. The most striking feature of Figure 15 is the enhancement of the photon flux upon the application of discrete microwave pulses. In essence, these results demonstrate the feasibility of increasing reaction rates of chemiluminescent reactions and dramatically improving photon flux for finite time intervals. As a result, chemiluminescent reactions that typically generate limited light emission over extended periods of time can be subsequently accelerated with the addition of low-power microwave pulses.

Significant enhancement with microwave pulses from silver island films was observed (data not shown). As compared to the

results of Figure 15 top, it was evident that there is a pronounced increase in photon flux from the metal surfaces; cf. Figure 15, top, a 3-fold enhancement in signal is observed from the silvered surfaces (data not shown). These results are further demonstrated with the insets in Figure 15 that show the real-color photographs of the chemiluminescent reagents (before and after Mw exposure) on glass and the SiF surfaces. When subjected to low-power microwaves (data not shown), chemiluminescence from the silver island films is even further enhanced for the microwave pulse time intervals. It is theorized that the high photon flux evident upon delivery of microwave pulses to the metal surface is attributed to localized heating of the metal surfaces. The local temperature increase not only accelerates the rate of the chemiluminescence reactions, but the proximity to the silver allows for metal-enhanced chemiluminescence. Thus, a reaction that traditionally is followed over extended periods of time can be "triggered" in short discrete time intervals with low-power microwaves.

The microwave heating of the whole sample (SiFs, HRP, and bulk solution) affects the enzyme-catalyzed chemiluminescence reactions in two ways: (1) since the enzyme is only on the surface of the silver nanoparticles, the chemiluminescence reactions only happen on SiFs, and the dissipated energy by SiFs is thought to lower the energy required for these reactions; (2) the heating of the solution increases the diffusion of chemiluminescent species so that the chemiluminescence reactions go faster. Although, the percent contribution of these factors to the overall reaction rate is unknown, it is believed that the localized heating effect is more dominant.

The chemiluminescent reactants and HRP-streptavidin were mixed in solution (100 $\mu$g/mL) to demonstrate that the localized chemiluminescent enhancement in the presence of silver island films is no longer observed. Data were collected for 400 s, and solutions were pulsed with low-power microwaves for 30 s at the 100- and 200-s time points during the course of the reaction. Figure 17, top and bottom, depict a fast signal decay for the reactions in solution above both glass and silver. In addition, upon application of the first microwave pulse, a small signal enhancement was evident, which is due to the few HRP molecules and chemiluminescent reactants that have settled close to the surfaces. For the second microwave pulse, very little signal enhancement is seen and, eventually, no signal observed at longer times. It is theorized that this result affirms the assertion that preferential heating of the nanostructures by microwaves affords for MT-MEC to be localized in proximity to the silvered surfaces, alleviating unwanted emission from the distal solution.

In order to demonstrate the "on-demand" nature of MT-MEC and induce the higher sensitivity of detection, the amount of biotinylated BSA incubated on the substrate surfaces was varied to demonstrate the concentration dependence for MT-MEC. Figure 18 shows the time-dependent chemiluminescent emission of the chemiluminescence reaction on SiFs and glass surfaces with multiple microwave exposures (four 30-s exposures, 100-s intervals). As previously observed in Figure 15, the intensity "spikes" correspond to the microwave pulses that trigger enhanced chemiluminescence from the HRP functionalized substrates. Each curve (a-e) corresponds to a different concentration of biotinylated BSA incubated on a silver substrate.

From Figure 18, it can be determined that the chemiluminescence intensity is proportional to the concentration of HRP bound to BSA-functionalized surfaces. Thus, this result, enables the surface protein concentration to be determined. It is important to explain the characteristics of the chemiluminescent intensity versus time plot, as shown in Figure 18. In order to determine the concentration of surface proteins without microwave heating, the change in chemiluminescent intensity was monitored after the chemiluminescent reactions were initiated (no microwave heating) in the first 100 s. It is seen that, without microwave heating, the chemiluminescent intensity is slightly increased as the concentration of BSA is increased but little difference between them is observed, which proved to be a not useful method. On the other hand, to show the benefits of microwave heating to increase the detected chemiluminescent signal, four 30-s exposures (after 100 s) were performed with 100-s intervals to drive the chemiluminescent reactions to completion within 400 s (without microwave heating the reactions studies here are completed longer than 30 min). The photon flux (in counts), area under the intensity-time plot, is an indication of the extent of the HRP-catalyzed reaction and thus provides information about the presence of surface-bound BSA. The two "peaks" seen after each microwave exposure, in Figure 18, are a result of the microwave magnetron pulsing. During the 10- and 5-s runs, the chemiluminescent intensity increases and decreases, respectively triggered by the magnetron pulsing and the localized heating of the microwaves. The peak height and the area under one of the peaks could be increased by using shorter exposure times (<10 s) and higher initial microwave power settings. However, it was found that higher initial power setting causes surface drying and was not found reliable for use here, as surface drying causes protein denaturation. In all the experiments performed with low-power microwaves, using both SiFs and glass, there was no evidence of surface drying. This is attributed to the previously made observations [20] that the temperature increase of the aqueous solution on the surfaces due to microwave heating is only ~8 ° C (to ~28 °C) for 30 $\mu$L of aqueous sample [20].

It is interesting to compare the results of the protein concentration-dependent assays on both silvered and glass surfaces, Figure 19. The overall signal enhancement shown in Figure 18, for assays performed on silver substrates versus those on glass substrates, serves to confirm the benefits of using silver nanostructures, for MEC. By combining the use of low-power microwaves and metal substrates to increase the rapidity of streptavidin binding to biotinylated BSA surfaces, decrease nonspecific background, and enhance and accelerate chemiluminescent reactions, Figure 19 shows that it is possible to detect approximately femtomoles of biotinylated BSA on surfaces in less than 2 min, with a signal-to-noise ratio (S/N) greater than 8. Signal-to-noise ratio is obtained from Figure 19, and is equal to the ratio of the lowest counts (y-axis) obtained using HRP divided by the counts without HRP (horizontal lines): for Ag, S/N = 7200/900 counts > 8.

As compared to traditional western blot approaches. Figure 20, MT-MEC offers protein quantification with ultrafast assay times, i.e., <2-min total assay time versus ~80 min.

3..8 Conclusions

Using low-power microwaves, it has been demonstrated as an inexpensive and simplistic approach to overcome some of the classical physical constraints imposed by current protein detection platforms, namely, assay rapidity, sensitivity, specificity, and accurate protein quantification. With the MT-MEC approach, the sensitivity of detection (<0.5 pg) is 1 order of magnitude greater than that available with currently standard commercially available methodologies (i.e., ECL Plus Western Blotting Detection Kit, RPN2132, Amersham Biosciences). In addition to the improved detection sensitivity, it is demonstrated herein that that these assays can be performed in a fraction of the time (in fact, less than 1 min) typically required with standard methodologies. With the application of microwaves and the subsequent acceleration of the chemiluminescent reaction, the on-demand nature of light emission not only increases the detectability of low concentrations of proteins, but photon flux is also proportional to the concentration of the protein on a surface. Thus, for immunoassays in the clinical setting, the MT-MEC approach offers a potentially powerful approach to protein detection because it substantially decreases current assay times to minutes, potentially decreases false positives due to increased specificity, and increases assay sensitivity by at least 1 order of magnitude (see Figure 19).

With the decreased reaction times, increased sensitivity, increased specificity, and signal enhancement achieved with MT-MBC, it is shown here a dramatically *decreased* the: volume of reagents required to perform these assays. Thus, by using MT-MEC into standard protein detection methodologies, reagent waste and overall experimental costs will be decreased. Further, with this technology, both ultrafast and ultra bright chemiluminescence assays can be realized.

References

All references cited herein are hereby incorporated by reference herein for all purposes.


[1] O. Hofmann, P. Miller, P. Sullivan, T.S. Jones, J.C. deMello, D.D.C. Bradley, A.J. deMello (2005). Thin-film organic photodiodes as integrated detectors for microscale chemiluminescence assays. Sensors and Actuators BChemical 106 (2), 878-884.

[2] O. Myhre, J.M. Andersen, H. Aarnes, F. Fonnum (2003). Evaluation of the probes 2',7'-dichlorofluorescin diacetate, luminol, and lucigenin as indicators of reactive species formation. Biochemical Pharmacology 65(10), 1575-1582.

[3] I. Bronstein, C.S. Martin, J.J. Fortin, C.E.M. Olesen, J.C. Voyta (1996). Chemiluminescence: Sensitive detection technology for reporter gene assays Clinical Chemistry 42 (9), 1542-1546.

[4] P. Moris, I. Alexandre, M. Roger, J. Remacle (1995). Chemiluminescence Assays of Organophosphorus and Carbamate Pesticides. Analytica Chimica Acta 302 (1), 53-59.

[5] Ä.M. Garcia-Campana, Willy R, Baeyens (2001). Chemiluminescence in Analytical Chemistry, Marcel Dekker. New York.

[6] J.E. Wampler (1985). Instrumentation: Seeing the Light and Measuring It, in Chemi- and Bioluminescence, J.G. Burr, Ed. Marcel Dekker, New York. pp. 1-44.

[7] F. Berthold (1990). Instrumentation for Chemilunescence Immunoassays, in Luminescence immunoassays and Molecular Applications, K. Van Dyke and R. Van Dyke, eds., CRC Press, Boca Raton, pp.11-25.

[8] T. Nieman (1995). Chemiluminescence: Theory and Instrumentation, Overview, in Encyclopedia of Analytical Science, Academic Press, Orlando, pp. 608-613.

[9] J.R. Lakowicz (1999). Principles of Fluorescence Spectroscopy, Kluwer, New York.

[10] K. Aslan, L Gryczynski, J. Malicka, E. Matveeva, J.R. Lakowicz, C.D. Geddes (2005). Metal-enhanced fluorescence: an emerging tool in biotechnology. Current Opinion in Biotechnology. 16(1), 55-62.

[11] K. Aslan, J.R, Lakowicz, C.D. Geddes (2005). Plasmon Light Scattering in Biology and Medicine: New Sensing Approaches, Visions and Perspectives, Current Opinions in Chemical Biology: Analytical Techniques, 9, 538-544.

[12] C.D. Geddes, K. Aslan, I. Gryczynski, J. Malicka, J.R. Lakowicz (2005). Radiative Decay Engineering, Review Chapter for Topics in Fluorescence Spectroscopy, Eds. C.D. Geddes and J.R. Lakowicz, Kluwer Academic / Plenum Publishers, New York, USA, pp. 405-448.

[13] C.D. Geddes, K. Aslan, I. Gryczynski, J. Malicka, E. Matveeva, J.R. Lakowicz (2005). In Topics in Fluorescence in Fluorescence Spectroscopy, C.D. Geddes, J.R. Lakowicz, Eds; Kluwer Academic/Plenum Publishers, New York, USA; pp. 401-448.

[14] J.R. Lakowicz, I. Gryczynski, J. Malicka, Z. Gryczynski, C.D. Geddes (2002). Enhanced and localized multiphoton excited fluorescence near metallic silver islands: metallic islands can increase probe photostability J.Fluoresc., 12, 299-302.

[15] C.D. Geddes, H. Cao, I. Gryczynski, Z. Gryczynski, J. Fang, and J.R. Lakowicz (2003). Metal-enhanced fluorescence due to silver colloids on a planar surface: Potential applications of Indocyanine green to in vivo imaging. J. Phys. Chem. A. 107, 3443-3449.

[16] K. Aslan, J.R. Lakowicz, C. D. Geddes (2005). Rapid deposition of triangular silver nanoplates on planar surfaces: Application to metalenhanced fluorescence. J. Phys. Chem. B. 109, 6247-6251.

[17] K. Aslan, Z. Leonenko, J.R. Lakowicz, C.D. Geddes (2005). Fast and slow deposition of silver nanorods on planar surfaces: Application to metalenhanced fluorescence J. Phys. Chem. B. 109(8), 3157-3162.

[18] C.D. Geddes, A. Parfenov, D. Roll, J. Fang, and J. R. Lakowicz (2003). Electrochemical and laser deposition of silver, for use in metal enhanced fluorescence. Langmuir 19, 6236-6241.

[19] K. Aslan, R. Badugu, J.R. Lakowicz, C.D. Geddes (2005). Metal-enhanced fluorescence from plastic substrates. J. Fluoresc., 15(2), 99-104.

[20] K. Aslan, C.D. Geddes. Microwave-Accelerated Metal-Enhanced Fluorescence (MAMEF): A New Platform Technology for Ultra Fast and Ultra Bright Assays. Anal. Chem. 77(24), 8057-8067.

[21] J.R. Lakowicz (2001). Radiative decay engineering: Biophysical and biomedical applications. Anal. Biochem. 298, 1-24.

[22] J.R. Lakowicz, C.D. Geddes, I. Gryczynski, J. Malicka, Z. Gryczynski, K. Aslan, J. Lukomska, E. Matveeva, J. Zhang, R. Badugu, J. Huang (2004). Advances in surface-enhanced fluorescence, J. Fluoresc., 14(4), 425-441.

[23] K. Aslan, Z. Leonenko, J.R. Lakowicz, C.D. Geddes (2005). Annealed Silver-Island Films for Applications in Metal-Enhanced Fluorescence: Interpretation in Terms of Radiating Plasmons, J. of Fluores., 15(5), 643-654.

[24] I. Gryczynski, J. Malicka, Z. Gryezynski, J.R. Lakowicz, (2004). Radiative Decay Engineering 4. Experimental studies of surface plasmon-coupled directional emission. Anal. Biochem., 324, 170-182.

[25] C.D. Geddes, I. Gryczynski, J. Malicka, Z. Gryczynski, J.R. Lakowicz, (2004). Directional surface plasmon coupled emission, J. Fluoresc., 14, 119-123,

[26] J. Yguerabide; W Yguerabide; (1998) Anal Biochem. 262,137-176.

[27] L. Rivas, S. Sanchez-Cortes, J.V. Garcia-Ramos and G. Morcillo G., (2001) Growth of Silver Colloidal Particles Obtained by Citrate Reduction to Increase the Ramen Enhancement Factor, Langmuir, 17(3), 574-577.

[28] N. Shirtcliffe, U. Nickel and S. Schneider, (1999) Reproducible preparation of silver, sols with small particle size using borohydride reduction: For use as nuclei fox preparation of large particles, J. Colloid Interface Sci., 211(1), 122-129.

[29] I. Pastoriza-Santos, and L.M> Liz-Marzan, (2000) Reduction of silver nanoparticles in DMF. Formation of monolayers and stable colloids, Pure Appl. Chem., 72(1-2), 83-90 (2000).

[30] Pastoriza-Santos, C. Serra-Rodriquez and L.M. Liz-Marzan, (2000) Self-assembly of silver particle monolayers on glass from Ag.sup.+ solutions in DMF, J. Colloid Interface Sci., 221(2), 236-241.

[31] R. Bright, M.D. Musick. and MJ. Natan, (1998) Preparation and characterization of Ag colloid monolayers, Langmuir, 14(20), 5695-5701.

[32]. F. Ni and T.M., (1986) Chemical procedure for preparing surface-enhanced Roman scattering active silver films, Anal. Chem., 58(14), 3159-5163.

[33] U. Krelbig, M. Gartz and A. Hilger, (1997) Mie resonances: Sensors for physical and chemical cluster interface properties, Ber. Bunsenges, Phys. Chem., 101(11), 1593-1604.

[34] R.G. Freeman, K.C, Grabar, K.J. Allison, R.M. Bright R., J.A. Davis, A.P. Guthrie, M.B. Hommer, M.A. Jackson, P.C. Smith, D.G Walter and M.J. Natan, (1995) Self-assembled metal colloid monolayers: An approach to SERS substrates, Science, 267, 1629-1632.

[35] K.C. Grabar, R.G Freeman, M.B. Hommer and M.J. Natan, (1995) Preparation and characterization of Au colloid monolayers, Anal. Chem., 67, 735-743.

[36] Lakowicz, J. R. Anal. Biochem. 2004, 324, 153-169.

[37] Aslan, K., Pérez-Luna, V. H. Langmuir 2002, 18, 6059-6065.

[38] Cormier, M. J.; Prichard, P. M. J Biol. Chem. 106.8, 243, 4706-4714,

[39] Methods in Enzymology, Vol. VLII, M. A. Deluca (Ed.), 1978.

## Claims

**1.** A method for measuring concentration of receptor-ligand binding complex in a test sample, the method comprising:

providing a metallic surface having positioned thereon a receptor molecule having affinity for a ligand of interest, wherein the metallic surface comprises metallic islands, metallic nanostructures or metallic colloids;
contacting the receptor molecule with the test sample suspected of comprising the ligand of interest, wherein the ligand of interest will bind to the receptor molecule to form a receptor-ligand complex;
contacting the receptor-ligand complex with a detector molecule having affinity for the ligand to form a receptor-ligand-detector complex, wherein the detector molecule comprises a chemiluminescent label, wherein the chemiluminescent label is positioned about 5 nm to about 200 nm from the metallic surfaces;
triggering the chemiluminescent label to induce a chemically electronically excited state to produce chemiluminescence that couples with metallic surface plasmons for excitement thereof;
irradiating at least the test sample with microwave energy, wherein the microwave energy has a power input of from about 100 to 150 watts; and
measuring the intensity of radiation emitted from at least the excited metallic surface plasmons.

**2.** The method according to claim 1, wherein the metallic surface is fabricated from a noble metal.

**3.** The method according to claim 2, wherein the noble metal is silver, gold, or platinum.

**4.** The method according to claim 1, wherein the chemiluminescent label is positioned about 5 nm to about 30 nm from the metallic surfaces.

**5.** The method according to claim 1, wherein the test sample comprises a biomolecule which comprises nucleic acids, aromatic carbon ring structures, NADH, FAD, amino acids, carbohydrates, steroids, flavins, proteins, DNA, RNA, oligonucleotides, peptide nucleic acids, fatty acids, glucose, vitamins, purines, pyrimidines, formycin, lipids, phytochrome, phytofluor, or antibodies.

**6.** The method according to claim 1, wherein measuring the intensity of the radiation includes emitted chemiluminescence energy.

7. The method of claim 1, wherein measuring the intensity of the radiation includes unpolarized emissions.

**Patentansprüche**

1. Ein Verfahren zur Messung der Konzentration von Rezeptor-Ligand-Bindungskomplex in einer Testprobe, wobei das Verfahren umfasst:

   Bereitstellen einer metallischen Oberfläche, auf der ein Rezeptormolekül positioniert ist, das eine Affinität für einen Liganden von Interesse aufweist, wobei die metallische Oberfläche metallische Inseln, metallische Nanostrukturen oder metallische Kolloide umfasst;
   Kontaktieren des Rezeptormoleküls mit der Testprobe, von der vermutet wird, dass sie den Liganden von Interesse umfasst, wobei der Ligand von Interesse an das Rezeptormolekül binden wird, um einen Rezeptor-Ligand-Komplex zu bilden;
   Kontaktieren des Rezeptor-Ligand-Komplexes mit einem Detektormolekül, das eine Affinität für den Liganden aufweist, um einen Reporter-Ligand-Detektor-Komplex zu bilden, wobei das Detektormolekül einen chemilumineszenten Marker umfasst, wobei der chemilumineszente Marker etwa 5 nm bis etwa 200 nm von den metallischen Oberflächen entfernt positioniert ist;
   Auslösen des chemilumineszenten Markers, um einen chemisch-elektronisch angeregten Zustand zu induzieren, um eine Chemilumineszenz zu erzeugen, die mit metallischen Oberflächenplasmonen zu deren Anregung koppelt;
   Bestrahlen von mindestens der Testprobe mit Mikrowellenenergie, wobei die Mikrowellenenergie eine Energiezufuhr von etwa 100 bis 150 Watt aufweist; und
   Messen der Intensität der von mindestens den angeregten metallischen Oberflächenplasmonen emittierten Strahlung.

2. Das Verfahren von Anspruch 1, wobei die Metalloberfläche aus einem Edelmetall hergestellt ist.

3. Das Verfahren von Anspruch 2, wobei das Edelmetall Silber, Gold oder Platin ist.

4. Das Verfahren von Anspruch 1, wobei der chemilumineszente Marker etwa 5 nm bis etwa 30 nm von den metallischen Oberflächen entfernt positioniert ist.

5. Das Verfahren von Anspruch 1, wobei die Testprobe ein Biomolekül umfasst, das Nukleinsäuren, aromatische Kohlenstoffringstrukturen, NADH, FAD, Aminosäuren, Kohlenhydrate, Steroide, Flavine, Proteine, DNA, RNA, Oligonukleotide, Peptidnukleinsäuren, Fettsäuren, Glucose, Vitamine, Purine, Pyrimidine, Formycin, Lipide, Phytochrom, Phytofluor oder Antikörper umfasst.

6. Das Verfahren von Anspruch 1, wobei das Messen der Intensität der Strahlung emittierte Chemilumineszenz-Energie umfasst.

7. Das Verfahren von Anspruch 1, wobei die Messung der Intensität der Strahlung unpolarisierte Emissionen umfasst.

**Revendications**

1. - Procédé pour mesurer la concentration de complexe de liaison récepteur-ligand dans un échantillon d'essai, le procédé comprenant :

   fournir une surface métallique ayant, positionnée sur celle-ci, une molécule de récepteur ayant une affinité pour un ligand d'intérêt, la surface métallique comprenant des îlots métalliques, des nanostructures métalliques ou des colloïdes métalliques ;
   mettre la molécule de récepteur en contact avec l'échantillon d'essai suspecté de comprendre le ligand d'intérêt, le ligand d'intérêt se liera à la molécule de récepteur pour former un complexe récepteur-ligand ;
   mettre le complexe récepteur-ligand en contact avec une molécule de détecteur ayant une affinité pour le ligand pour former un complexe récepteur-ligand-détecteur, la molécule de détecteur comprenant une étiquette chimioluminescente, l'étiquette chimioluminescente étant positionnée entre environ 5 nm et environ 200 nm des surfaces métalliques ;
déclencher l'étiquette chimioluminescente pour induire un état d'excitation chimico-électronique pour produire une chimioluminescence qui se couple à des plasmons de surface métalliques pour une excitation de ceux-ci ;
irradier au moins l'échantillon d'essai avec de l'énergie micro-onde, l'énergie micro-onde ayant une puissance d'entrée comprise entre environ 100 et 150 watts ; et
mesurer l'intensité de rayonnement émis à partir d'au moins les plasmons de surface métalliques excités.

**2.** - Procédé selon la revendication 1, dans lequel la surface métallique est fabriquée à partir d'un métal noble.

**3.** - Procédé selon la revendication 2, dans lequel le métal noble est l'argent, l'or ou le platine.

**4.** - Procédé selon la revendication 1, dans lequel l'étiquette chimioluminescente est positionnée entre environ 5 nm et environ 30 nm des surfaces métalliques.

**5.** - Procédé selon la revendication 1, dans lequel l'échantillon d'essai comprend une biomolécule qui comprend des acides nucléiques, des structures de noyau carboné aromatique, NADH, FAD, des acides aminés, des hydrates de carbone, des stéroïdes, des flavines, des protéines, de l'ADN, de l'ARN, des oligonucléotides, des acides nucléiques peptidiques, des acides gras, du glucose, des vitamines, des purines, des pyrimidines, une formycine, des lipides, un phytochrome, un phytofluor ou des anticorps.

**6.** - Procédé selon la revendication 1, dans lequel mesurer l'intensité du rayonnement comprend de l'énergie de chimioluminescence émise.

**7.** - Procédé selon la revendication 1, dans lequel mesurer l'intensité du rayonnement comprend des émissions non-polarisées.

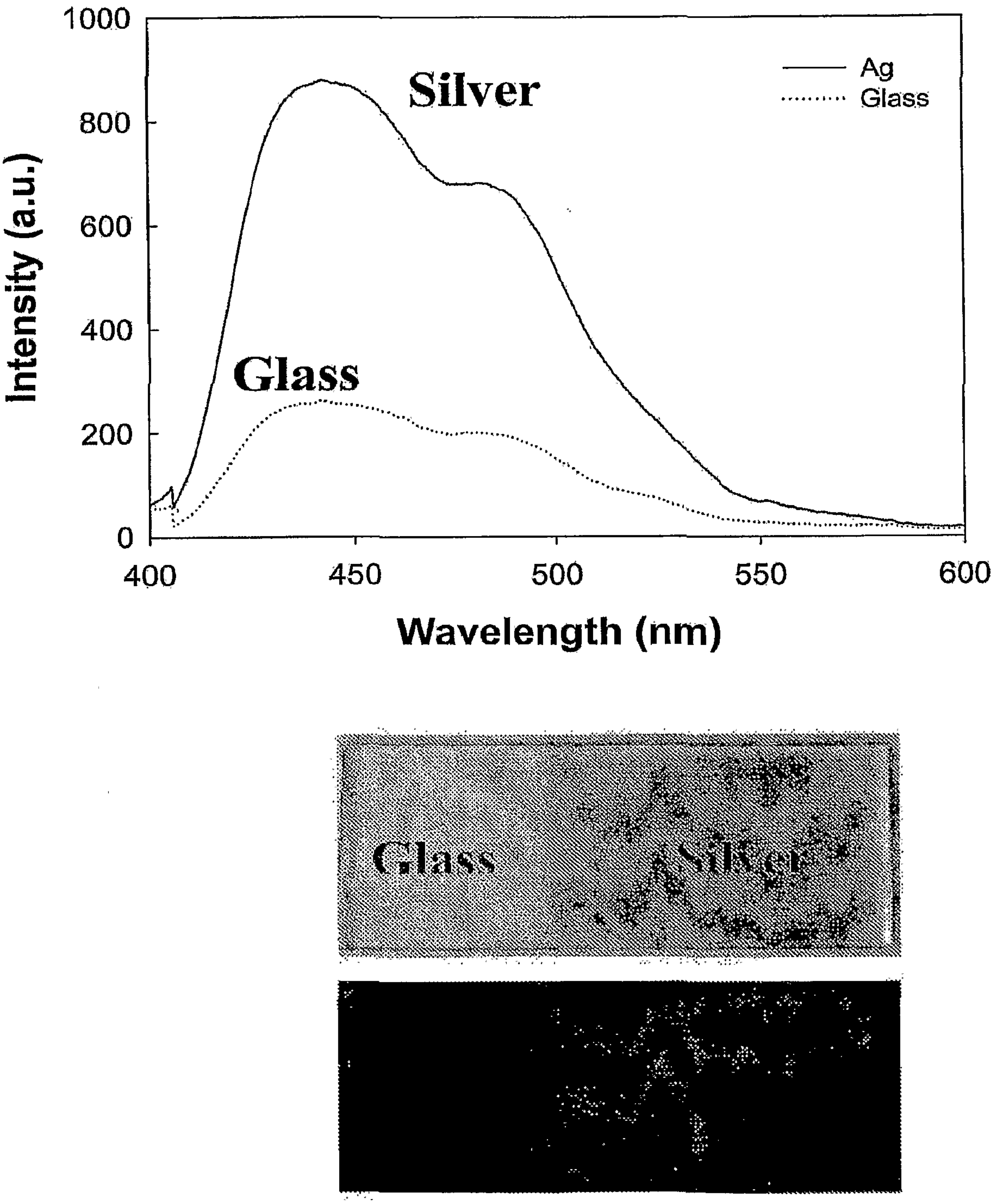
1000
800
600
400
200
0
Intensity (a.u.)
400
450
500
550
600
Wavelength (nm)
Silver
Glass
Ag
Glass
Glass
Silver

**Figure 1**

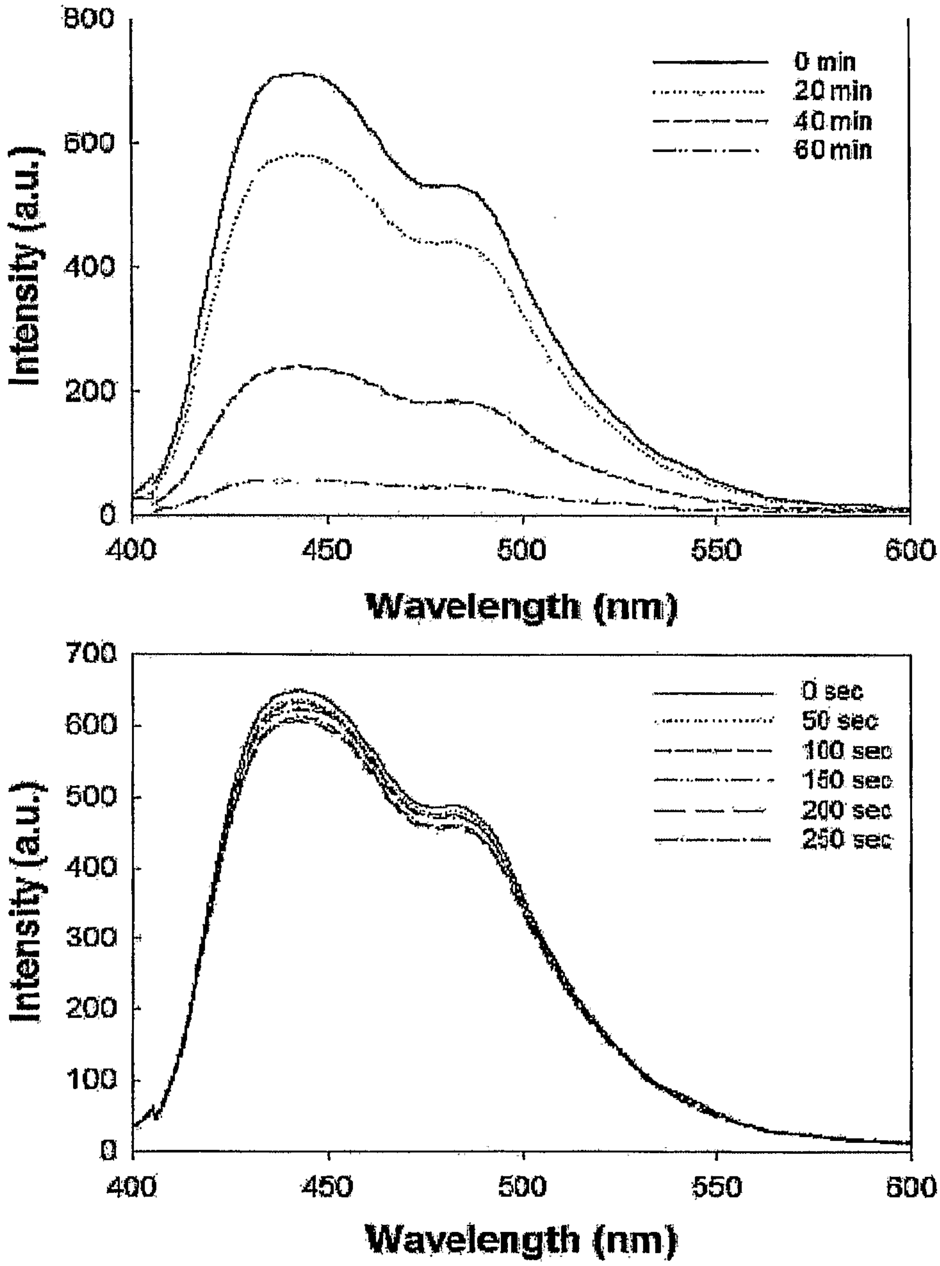
800
600
400
200
0
Intensity (a.u.)
400
450
500
550
600
Wavelength (nm)
0 min
20 min
40 min
60 min
700
600
500
400
300
200
100
0
Intensity (a.u.)
400
450
500
550
600
Wavelength (nm)
0 sec
50 sec
100 sec
150 sec
200 sec
250 sec

Figure 2

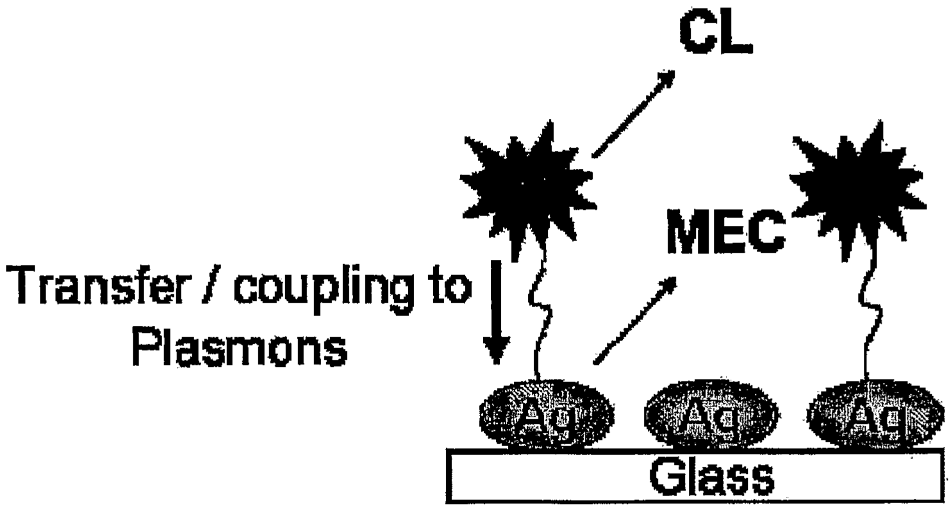


Figure 3

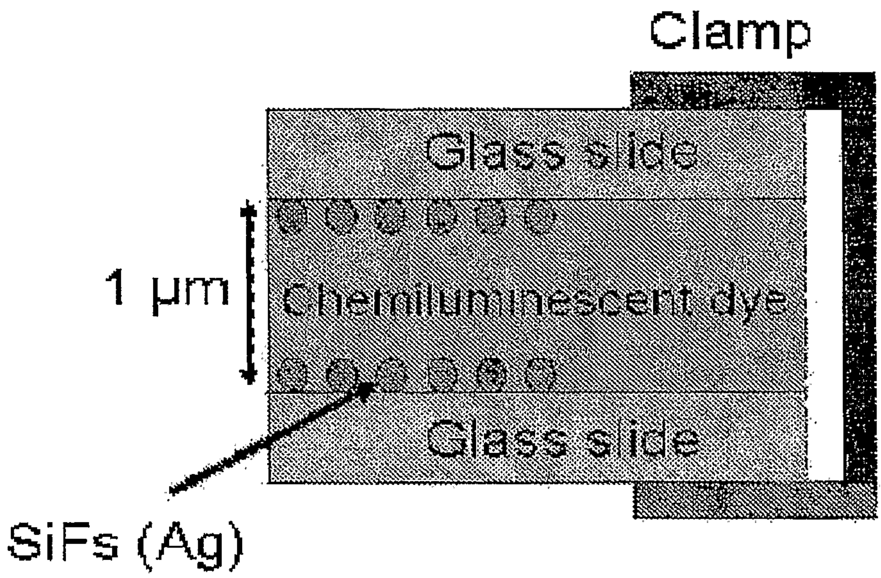
Clamp
Glass slide
1 µm
Chemiluminescent dye
Glass slide
SiFs (Ag)

**Figure 4**

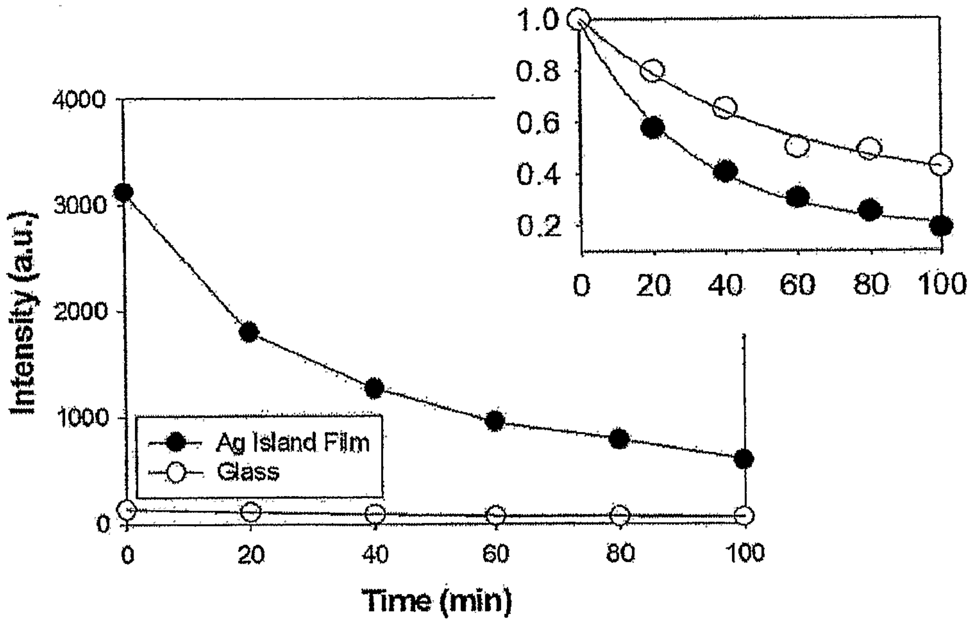
1.0
0.8
0.6
0.4
0.2
0
20
40
60
80
100
4000
3000
2000
1000
0
Intensity (a.u.)
Ag Island Film
Glass
Time (min)

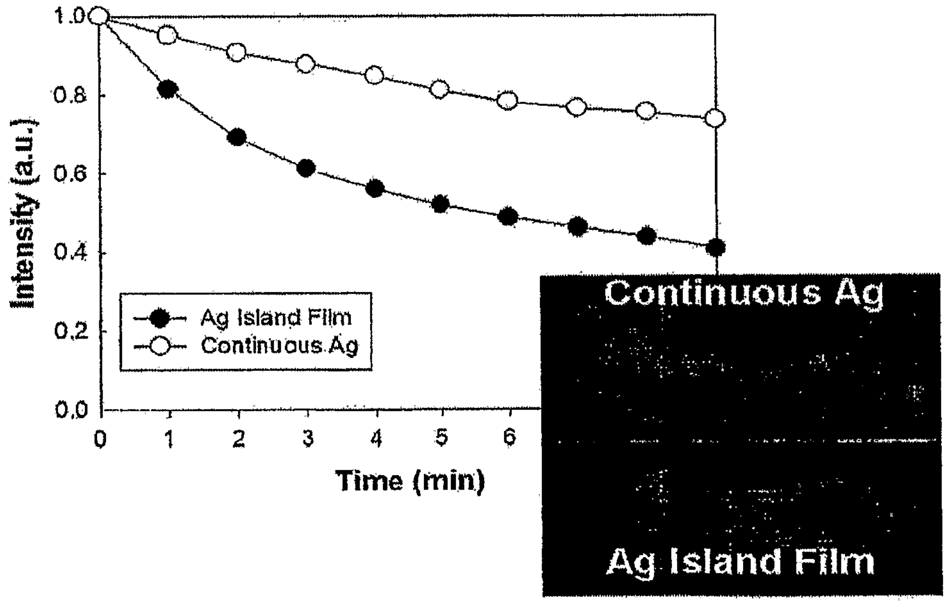
1.0
0.8
0.6
0.4
0.2
0.0
0
1
2
3
4
5
6
Intensity (a.u.)
Ag Island Film
Continuous Ag
Time (min)
Continuous Ag
Ag Island Film

# Figure 5

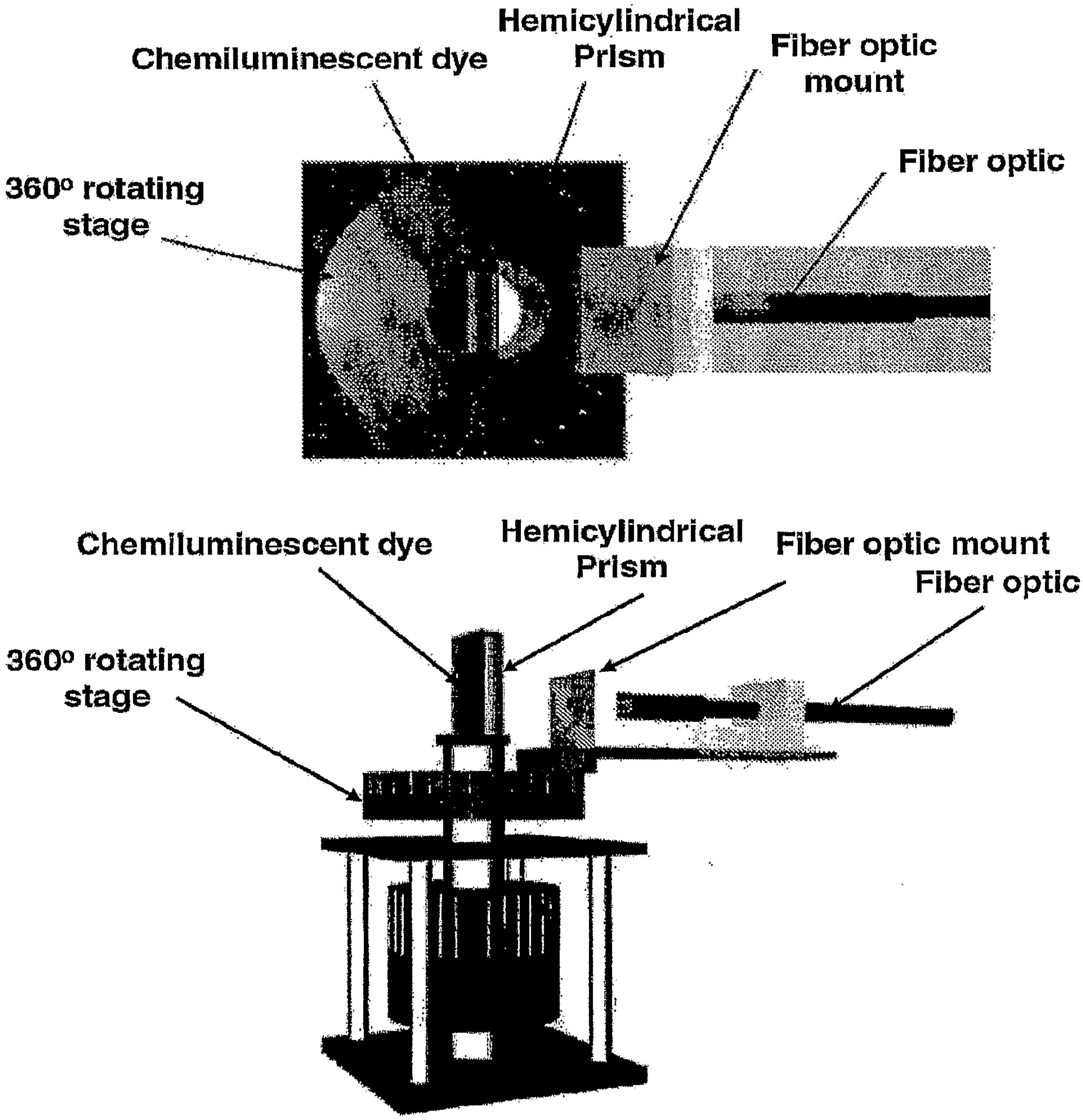
Chemiluminescent dye
Hemicylindrical Prism
Fiber optic mount
Fiber optic
360° rotating stage
Chemiluminescent dye
Hemicylindrical Prism
Fiber optic mount
Fiber optic
360° rotating stage

**Figure 6**

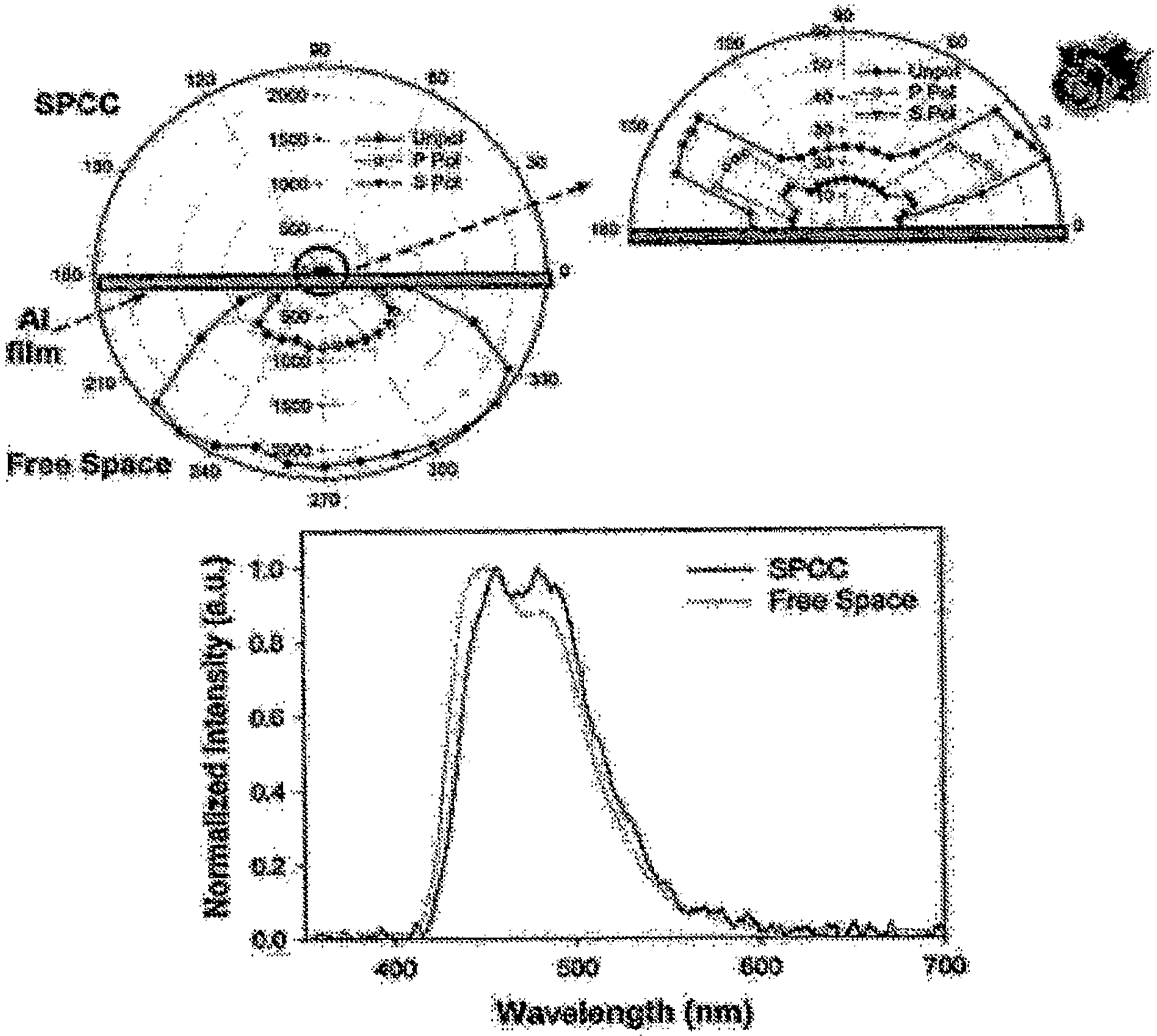
SPCC
Al film
Free Space
Unpol
P Pol
S Pol
SPCC
Free Space
Normalized Intensity (a.u.)
Wavelength (nm)

# Figure 7

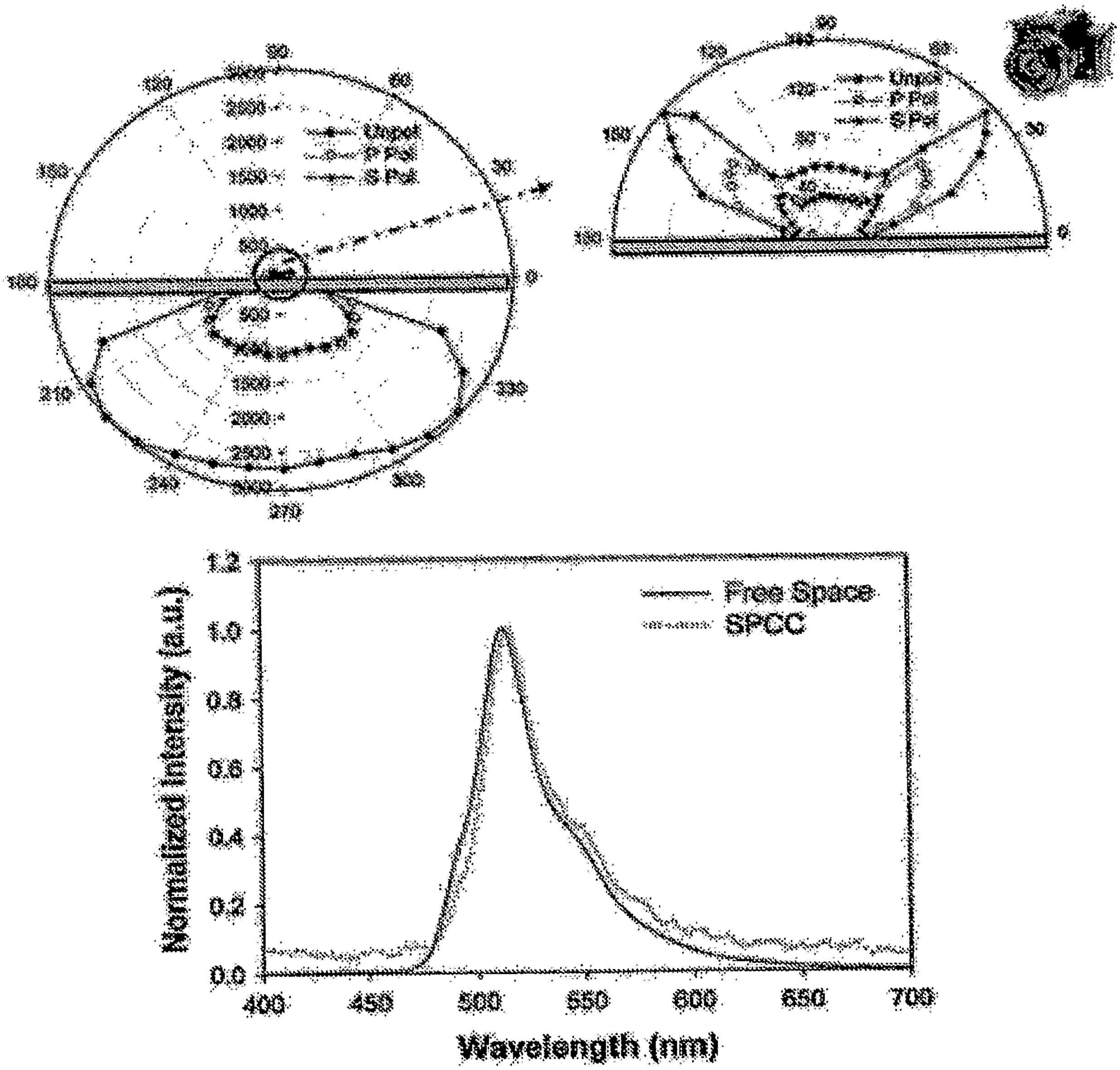
Unpol
P Pol
S Pol
Free Space
SPCC
Normalized Intensity (a.u.)
Wavelength (nm)
0.0
0.2
0.4
0.6
0.8
1.0
1.2
400
450
500
550
600
650
700

Figure 8

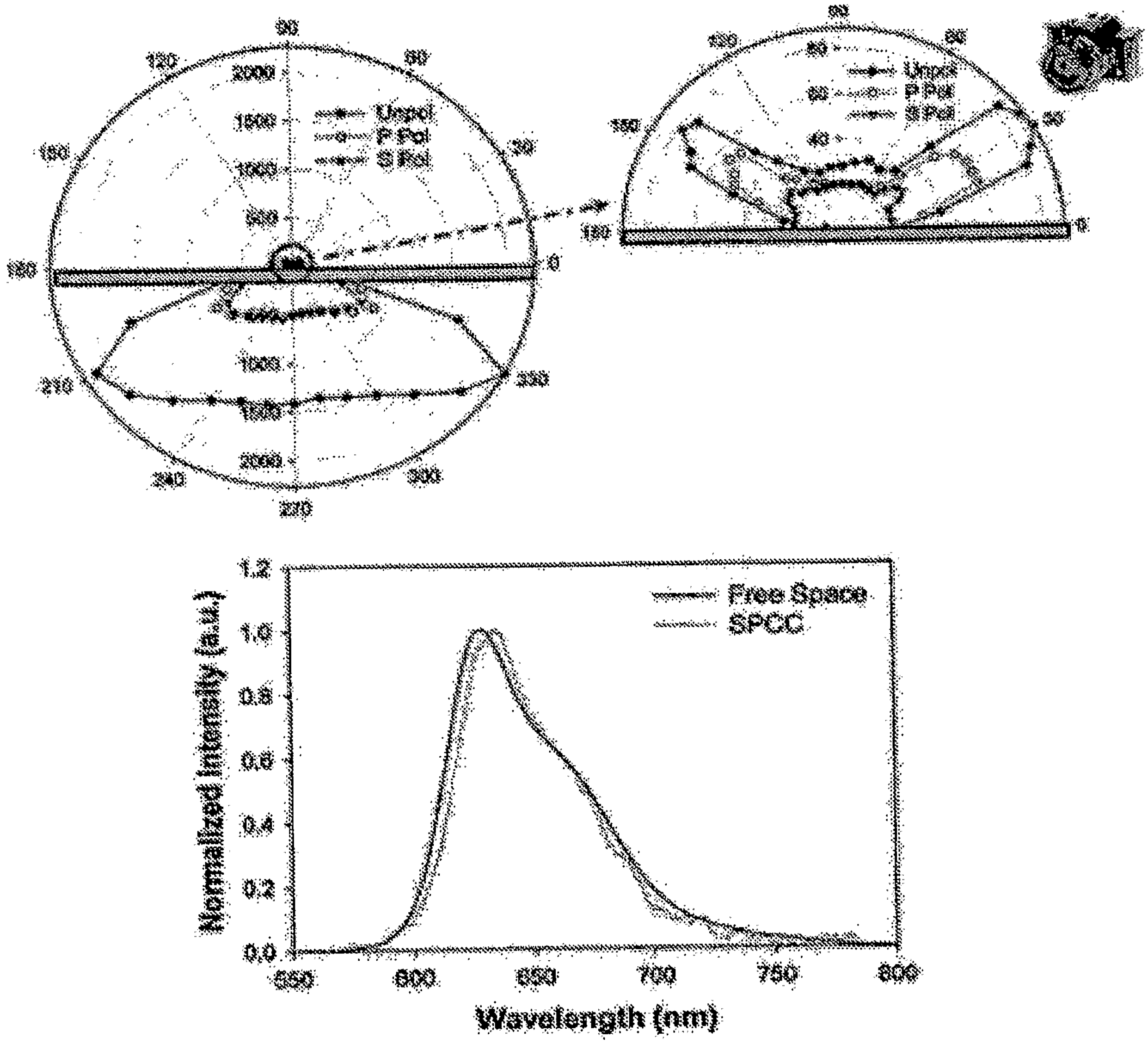
Unpol
P Pol
S Pol
Free Space
SPCC
Normalized Intensity (a.u.)
Wavelength (nm)

**Figure 9**

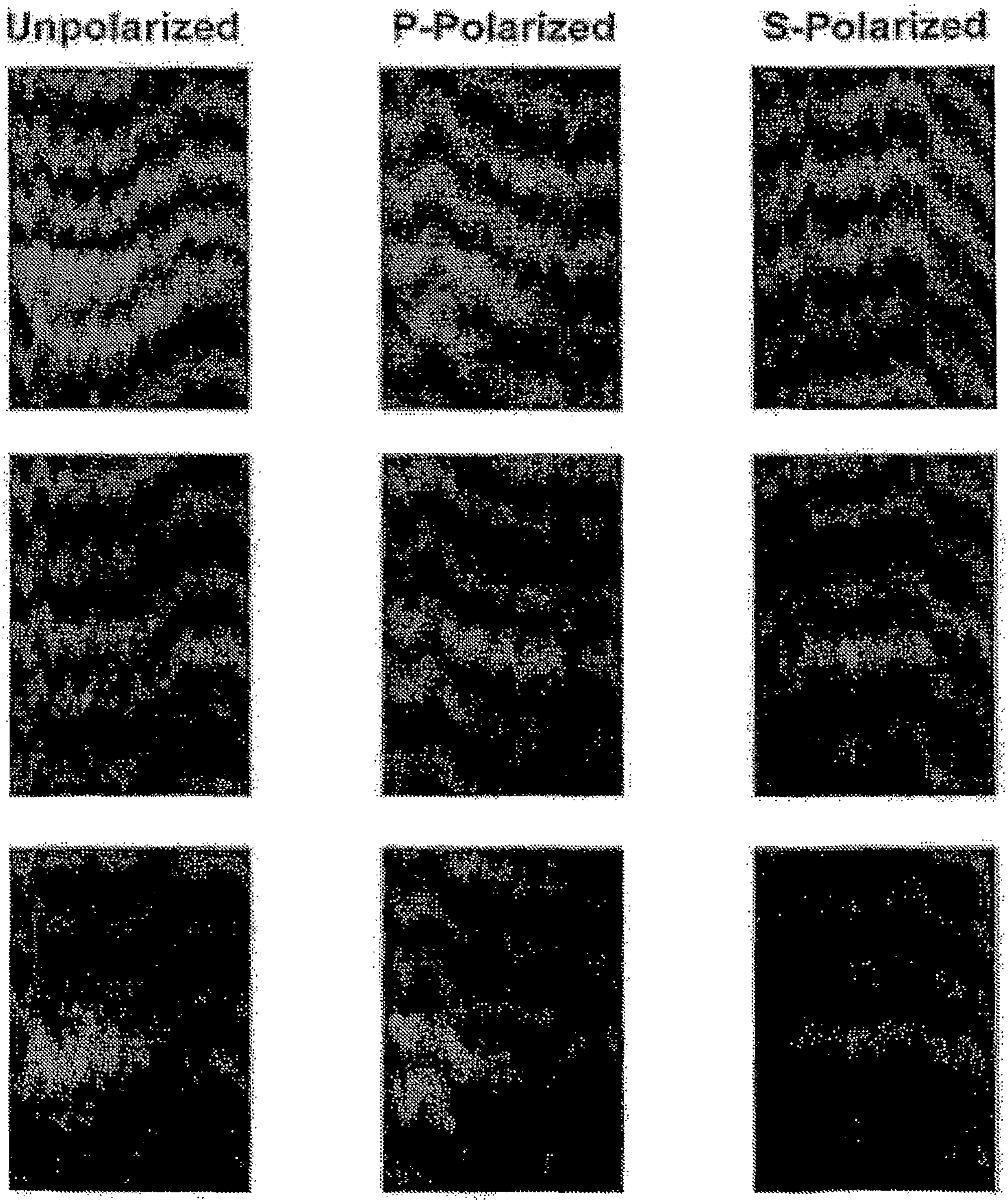


**Figure 10**

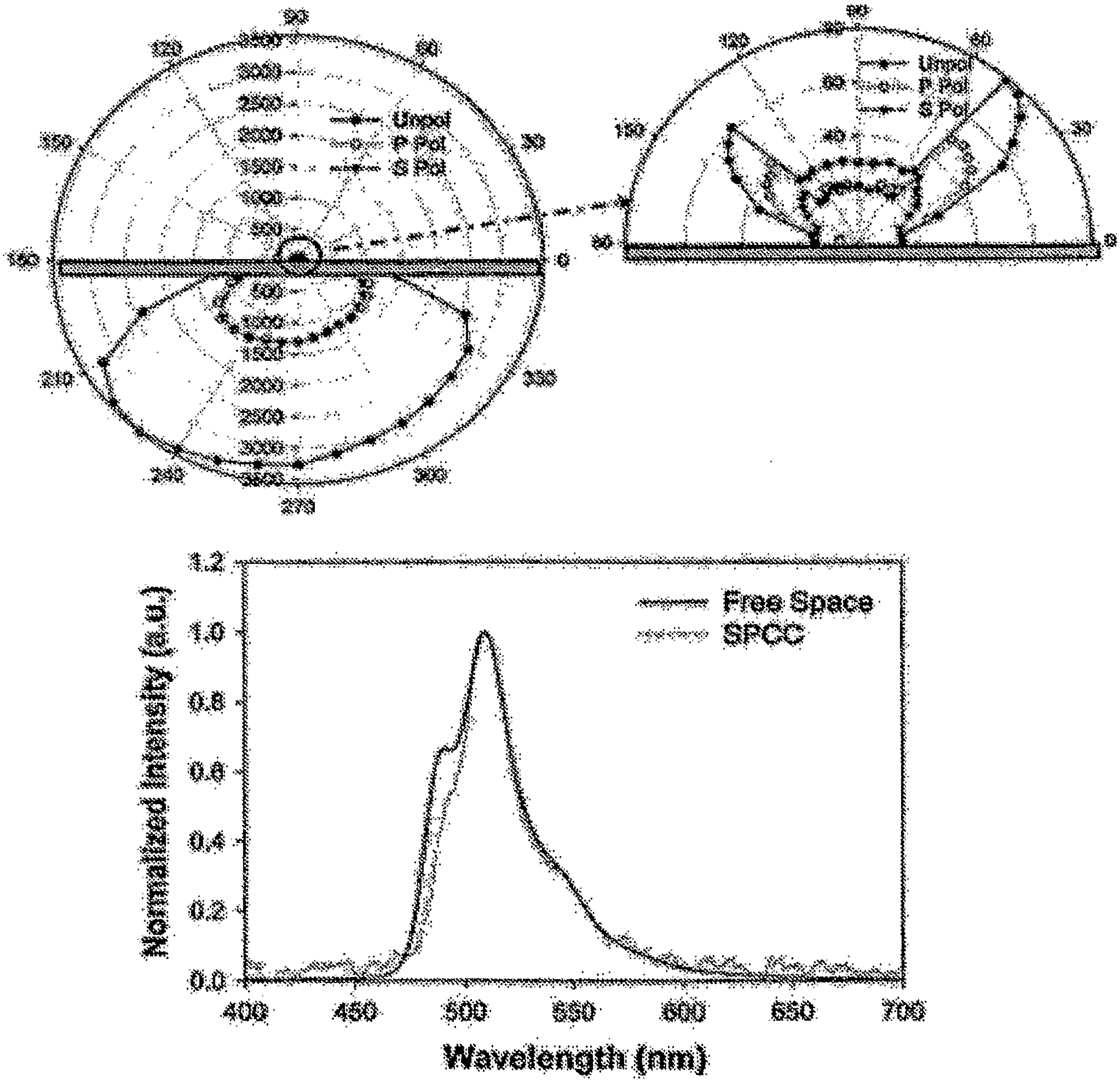
Unpol
P Pol
S Pol
Free Space
SPCC
Normalized Intensity (a.u.)
Wavelength (nm)

Figure 11

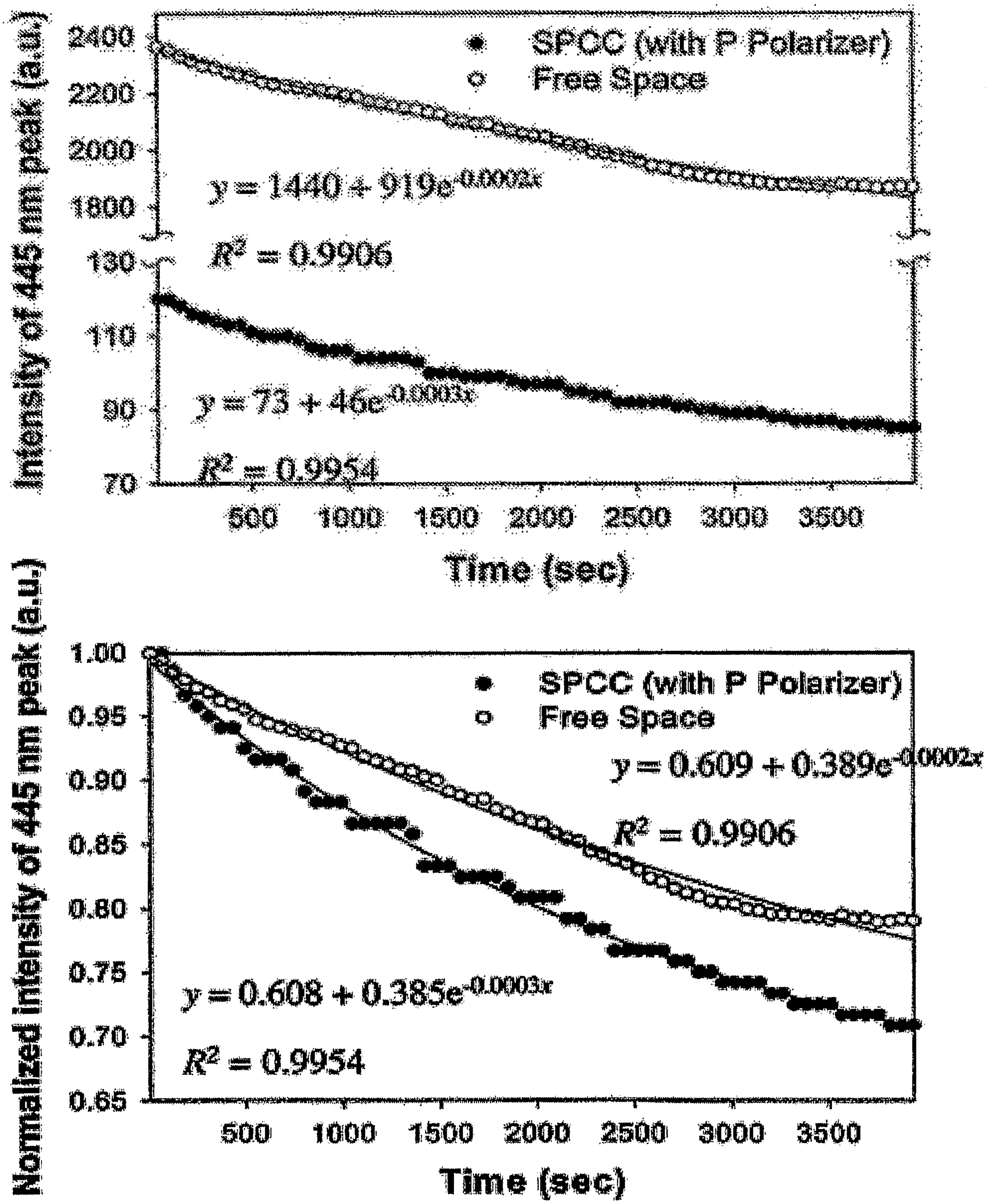
Intensity of 445 nm peak (a.u.)
2400
2200
2000
1800
130
110
90
70
SPCC (with P Polarizer)
Free Space
y = 1440 + 919e^-0.0002x
R² = 0.9906
y = 73 + 46e^-0.0003x
R² = 0.9954
500 1000 1500 2000 2500 3000 3500
Time (sec)
Normalized intensity of 445 nm peak (a.u.)
1.00
0.95
0.90
0.85
0.80
0.75
0.70
0.65
SPCC (with P Polarizer)
Free Space
y = 0.609 + 0.389e^-0.0002x
R² = 0.9906
y = 0.608 + 0.385e^-0.0003x
R² = 0.9954
500 1000 1500 2000 2500 3000 3500
Time (sec)

Figure 12

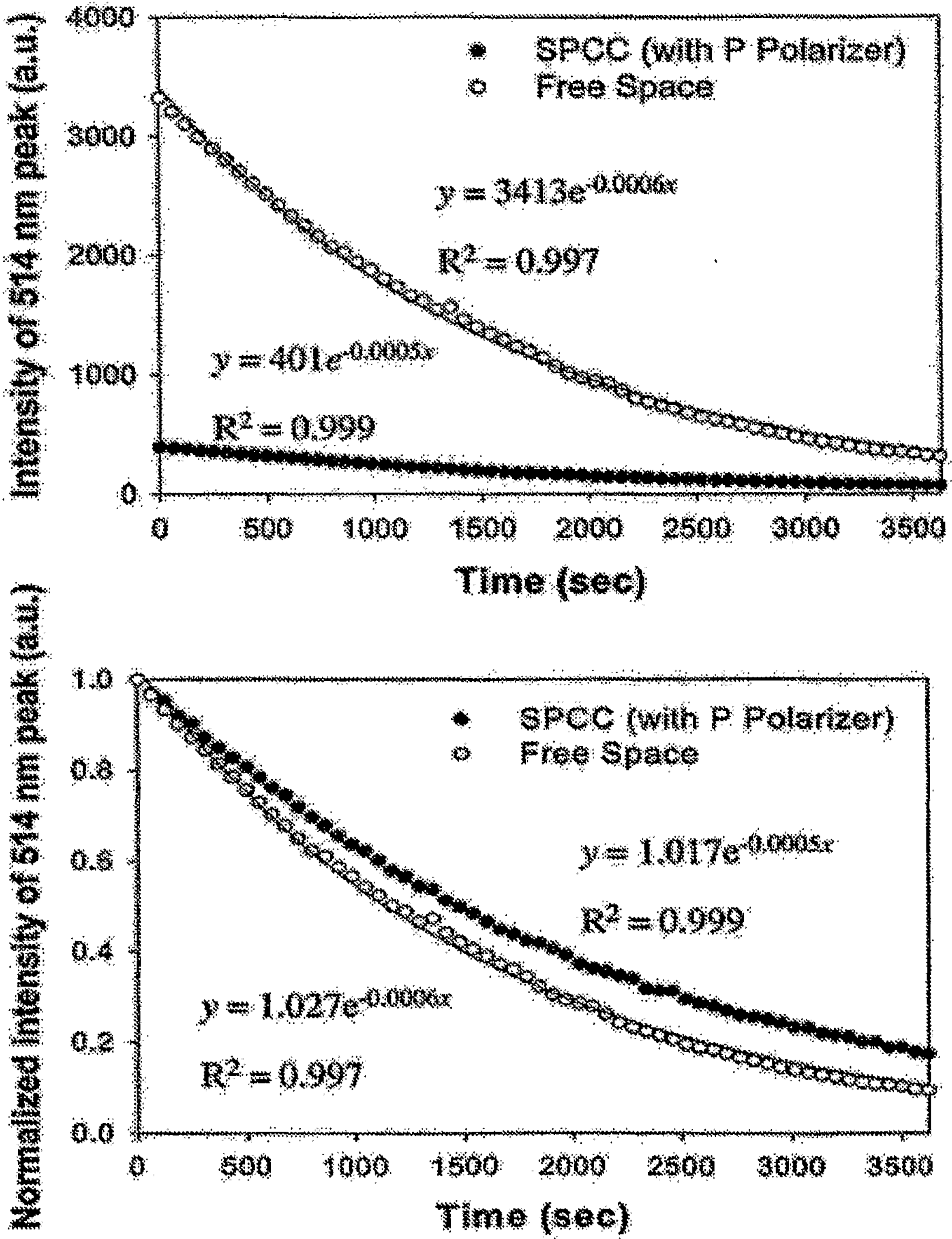
Intensity of 514 nm peak (a.u.)
4000
3000
2000
1000
0
SPCC (with P Polarizer)
Free Space
y = 3413e^-0.0006x
R² = 0.997
y = 401e^-0.0005x
R² = 0.999
0 500 1000 1500 2000 2500 3000 3500
Time (sec)
Normalized intensity of 514 nm peak (a.u.)
1.0
0.8
0.6
0.4
0.2
0.0
SPCC (with P Polarizer)
Free Space
y = 1.017e^-0.0005x
R² = 0.999
y = 1.027e^-0.0006x
R² = 0.997
0 500 1000 1500 2000 2500 3000 3500
Time (sec)

Figure 13

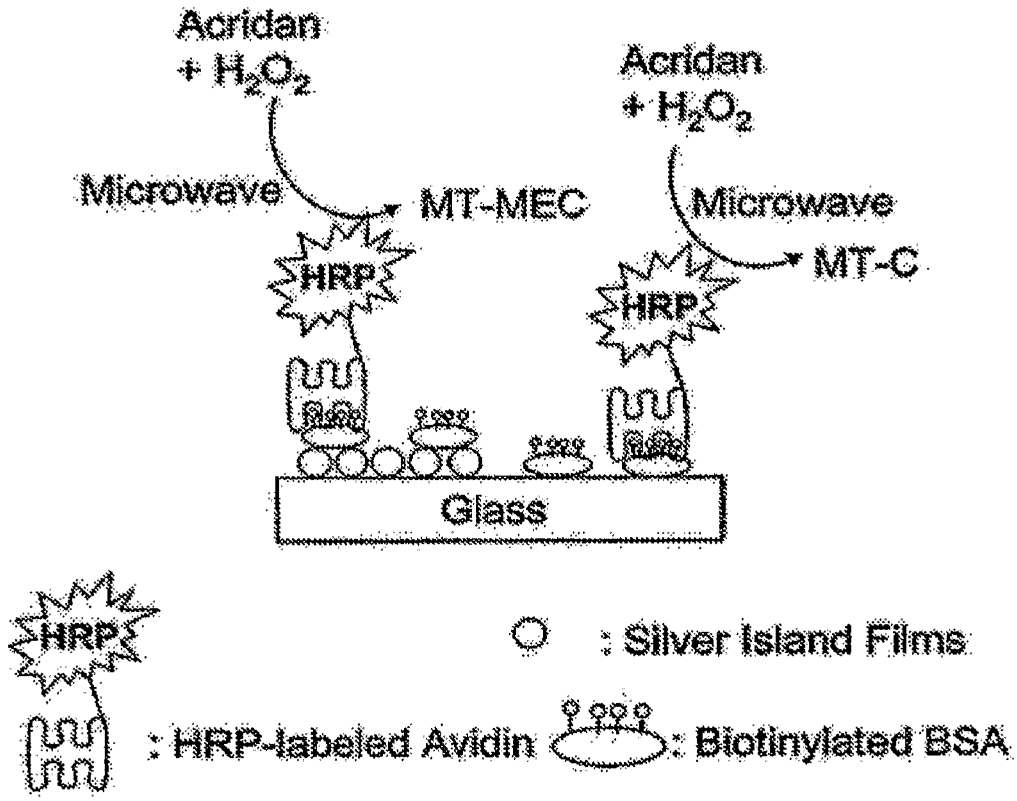
Acridan
+ $H_2O_2$
Microwave
MT-MEC
HRP
Acridan
+ $H_2O_2$
Microwave
MT-C
HRP
Glass
HRP
: Silver Island Films
: HRP-labeled Avidin
: Biotinylated BSA

Figure 14

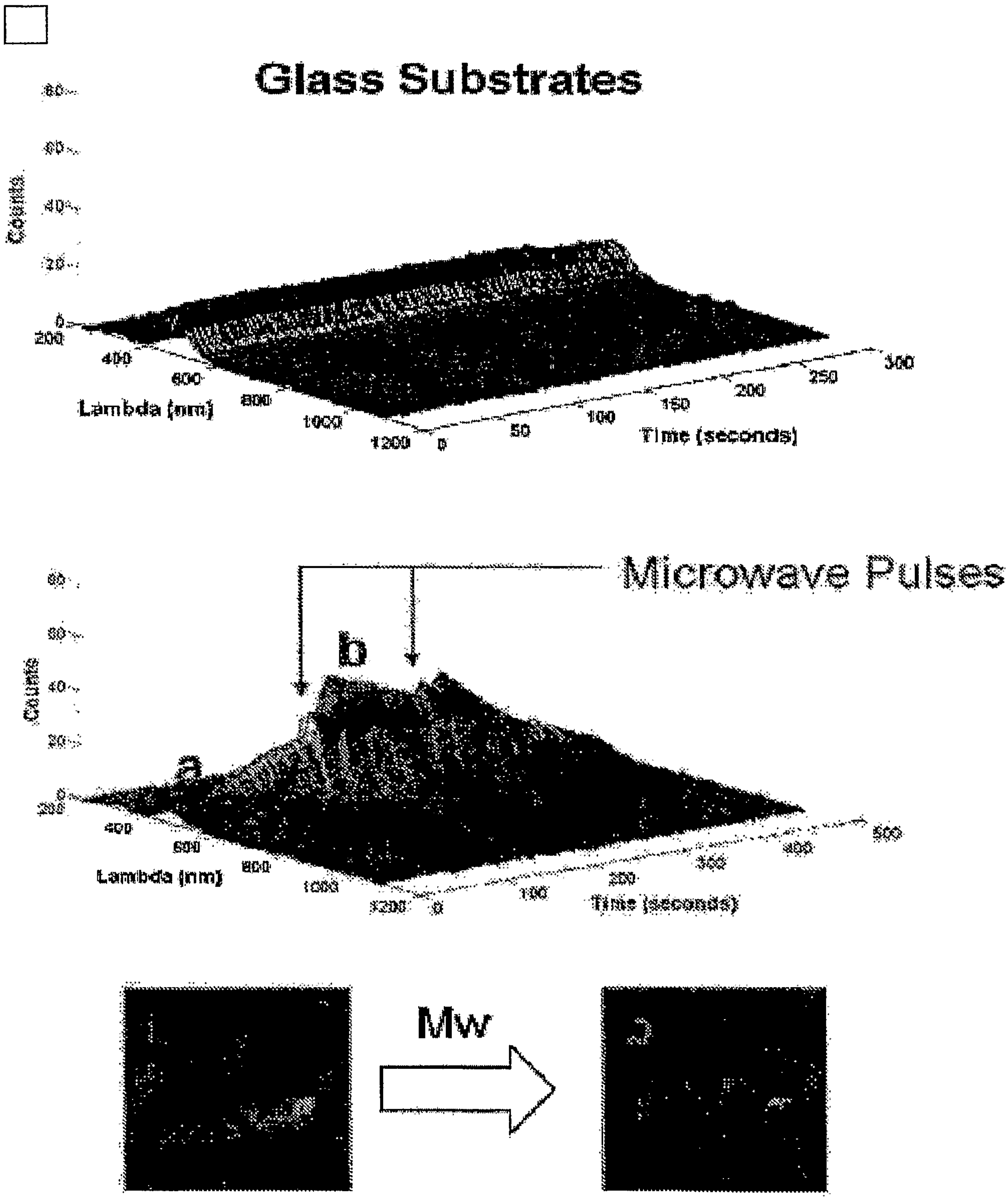
Glass Substrates
Counts
Lambda (nm)
Time (seconds)
Microwave Pulses
b
a
Counts
Lambda (nm)
Time (seconds)
Mw

Figure 15

Fig. 16 missing

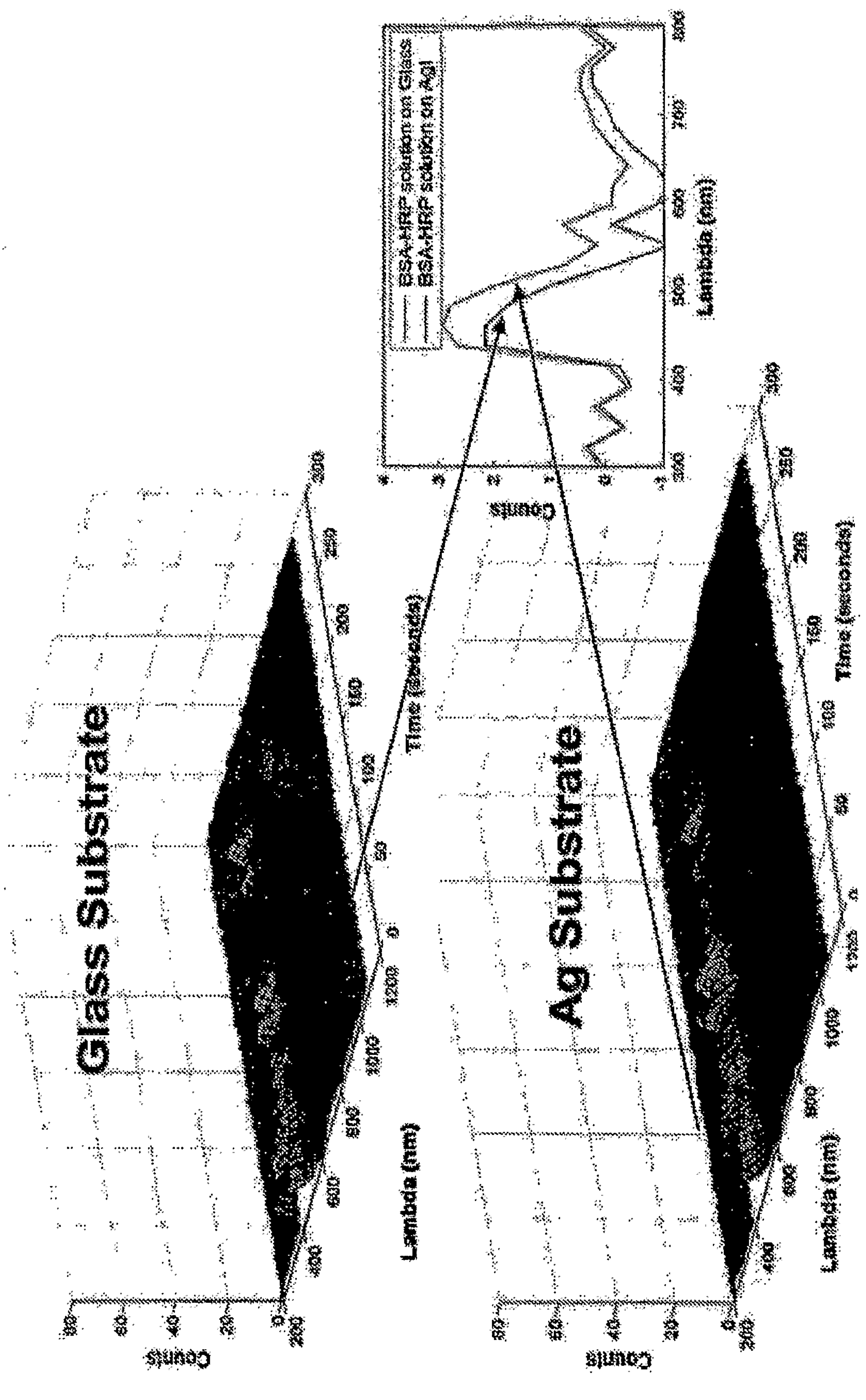
Glass Substrate
Ag Substrate
Counts
Lambda (nm)
Time (seconds)
BSA-HRP solution on Glass
BSA-HRP solution on Ag

Figure 17

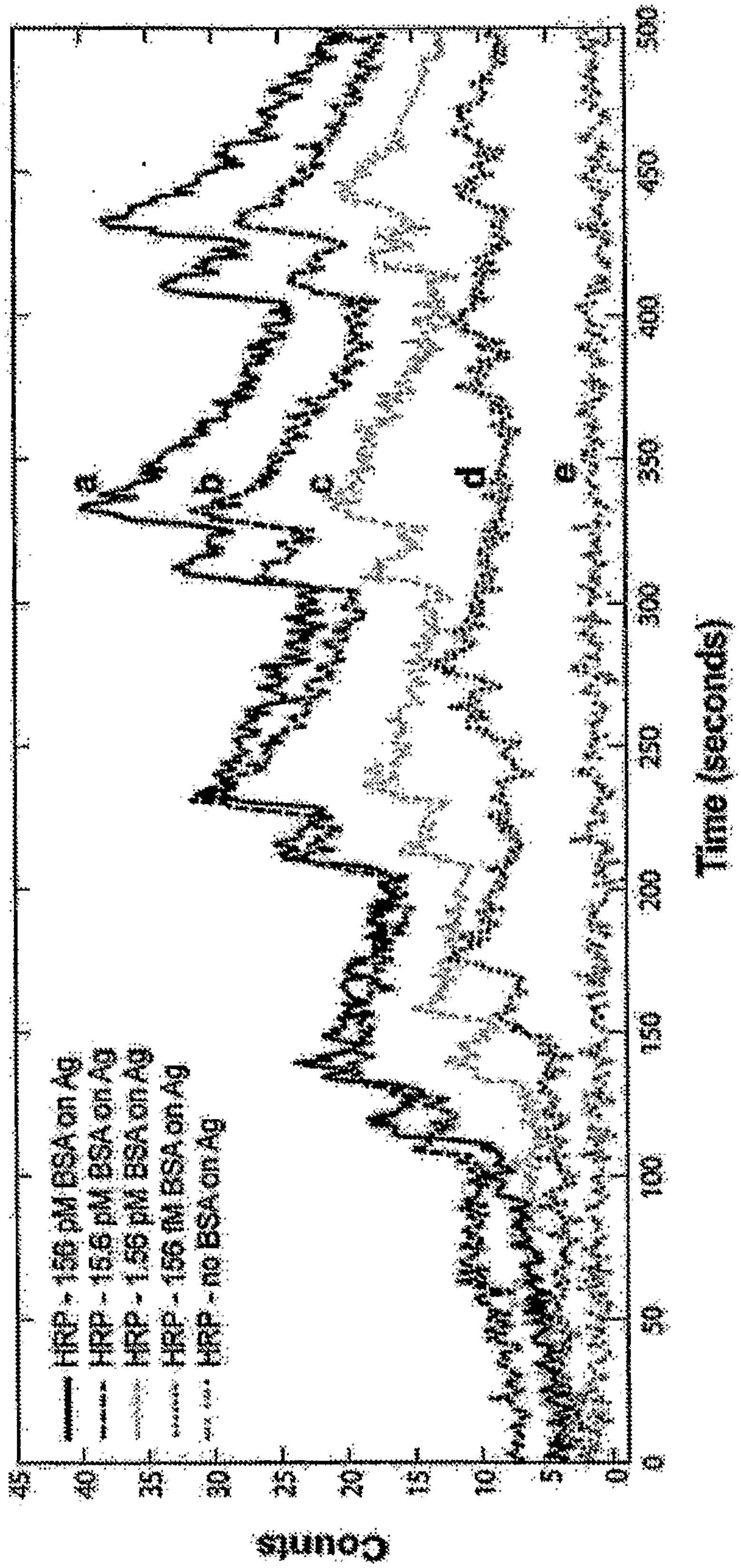
HRP - 156 pM BSA on Ag
HRP - 15.6 pM BSA on Ag
HRP - 1.56 pM BSA on Ag
HRP - 156 fM BSA on Ag
HRP - no BSA on Ag
a
b
c
d
e
Counts
Time (seconds)
0
5
10
15
20
25
30
35
40
45
0
50
100
150
200
250
300
350
400
450
500

Figure 18

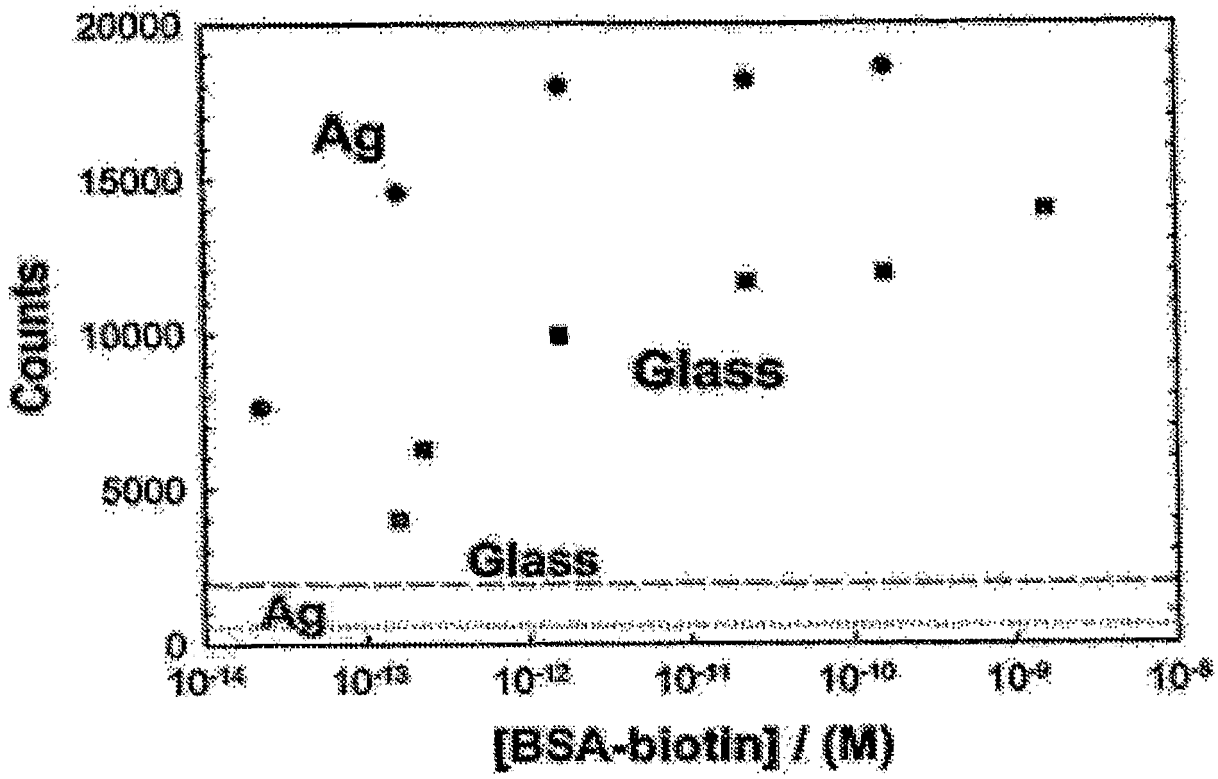

20000
15000
10000
5000
0
Counts
Ag
Glass
Glass
Ag
10^-14
10^-13
10^-12
10^-11
10^-10
10^-9
10^-8
[BSA-biotin] / (M)


Figure 19

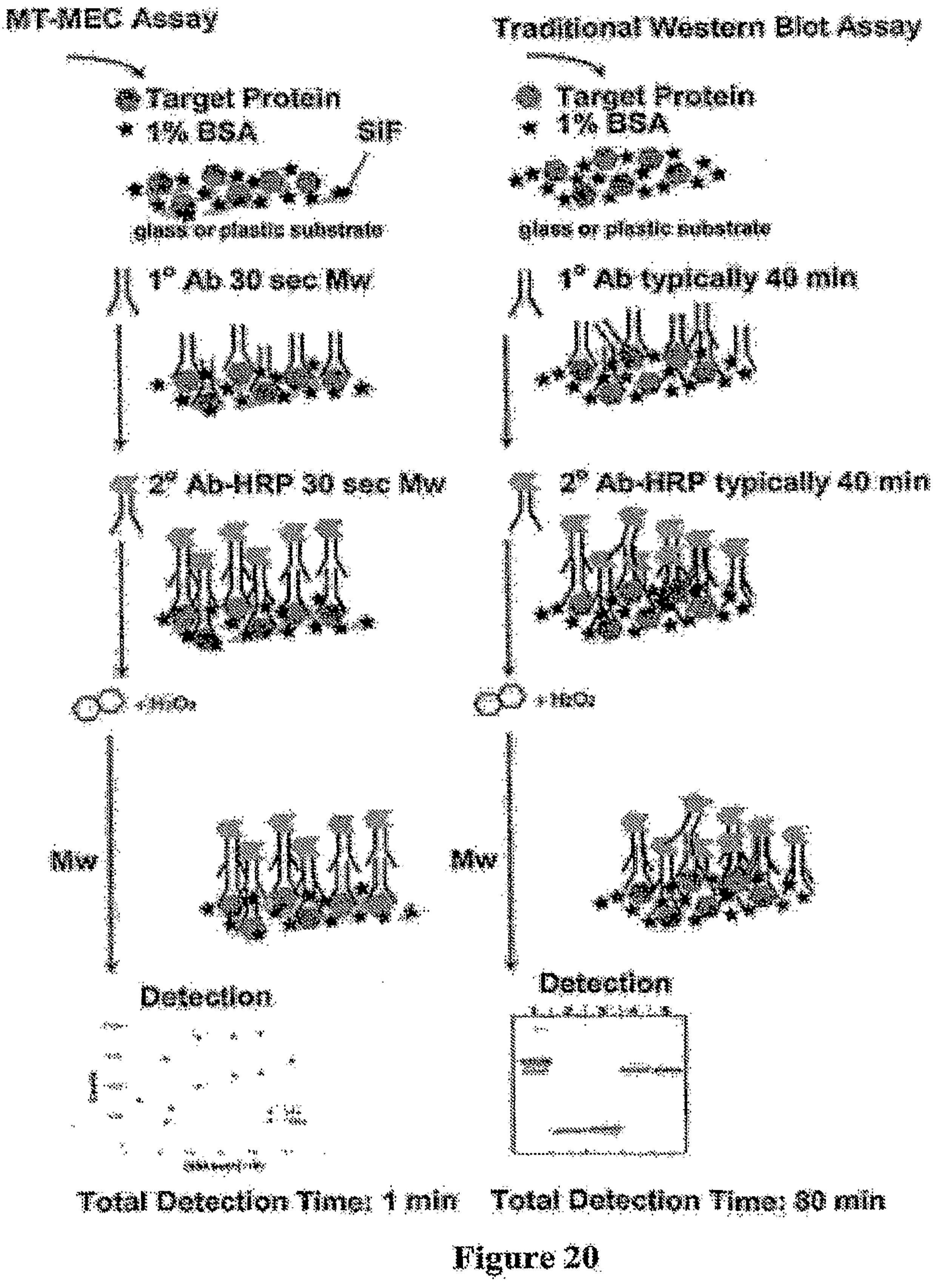
MT-MEC Assay
Traditional Western Blot Assay
Target Protein
1% BSA
SIF
glass or plastic substrate
Target Protein
1% BSA
glass or plastic substrate
1° Ab 30 sec Mw
1° Ab typically 40 min
2° Ab-HRP 30 sec Mw
2° Ab-HRP typically 40 min
Mw
Mw
Detection
Detection
Total Detection Time: 1 min
Total Detection Time: 80 min

**Figure 20**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20030082633 A

### Non-patent literature cited in the description


- **JIAN ZHANG et al.** First Observation of Surface Plasmon-Coupled Electrochemiluminescence. *Chemical Physics Letters,* 01 August 2004, vol. 393 (4-6), 483-487
- **O. HOFMANN ; P. MILLER ; P. SULLIVAN ; T.S. JONES ; J.C. DEMELLO ; D.D.C. BRADLEY ; A.J. DEMELLO.** Thin-film organic photodiodes as integrated detectors for microscale chemiluminescence assays. *Sensors and Actuators BChemical,* 2005, vol. 106 (2), 878-884
- **O. MYHRE ; J.M. ANDERSEN ; H. AARNES ; F. FONNUM.** Evaluation of the probes 2',7'-dichlorofluorescin diacetate, luminol, and lucigenin as indicators of reactive species formation. *Biochemical Pharmacology,* 2003, vol. 65 (10), 1575-1582
- **I. BRONSTEIN ; C.S. MARTIN ; J.J. FORTIN ; C.E.M. OLESEN ; J.C. VOYTA.** Chemiluminescence: Sensitive detection technology for reporter gene assays. *Clinical Chemistry,* 1996, vol. 42 (9), 1542-1546
- **P. MORIS ; I. ALEXANDRE ; M. ROGER ; J. REMACLE.** Chemiluminescence Assays of Organophosphorus and Carbamate Pesticides. *Analytica Chimica Acta,* 1995, vol. 302 (1), 53-59
- **Ä.M. GARCIA-CAMPANA ; WILLY R, BAEYENS.** Chemiluminescence in Analytical Chemistry. Marcel Dekker, 2001
- Instrumentation: Seeing the Light and Measuring It. **J.E. WAMPLER.** Chemi- and Bioluminescence. Marcel Dekker, 1985, 1-44
- Instrumentation for Chemilunescence Immunoassays. **F. BERTHOLD.** Luminescence immunoassays and Molecular Applications. CRC Press, 1990, 11-25
- Chemiluminescence: Theory and Instrumentation, Overview. **T. NIEMAN.** Encyclopedia of Analytical Science. Academic Press, 1995, 608-613
- **J.R. LAKOWICZ.** Principles of Fluorescence Spectroscopy. Kluwer, 1999
- **K. ASLAN ; L GRYCZYNSKI ; J. MALICKA ; E. MATVEEVA ; J.R. LAKOWICZ ; C.D. GEDDES.** Metal-enhanced fluorescence: an emerging tool in biotechnology. *Current Opinion in Biotechnology,* 2005, vol. 16 (1), 55-62
- **K. ASLAN ; J.R, LAKOWICZ ; C.D. GEDDES.** Plasmon Light Scattering in Biology and Medicine: New Sensing Approaches, Visions and Perspectives. *Current Opinions in Chemical Biology: Analytical Techniques,* 2005, vol. 9, 538-544
- **C.D. GEDDES ; K. ASLAN ; I. GRYCZYNSKI ; J. MALICKA ; J.R. LAKOWICZ.** Radiative Decay Engineering, Review Chapter for Topics in Fluorescence Spectroscopy. Kluwer Academic / Plenum Publishers, 2005, 405-448
- **C.D. GEDDES ; K. ASLAN ; I. GRYCZYNSKI ; J. MALICKA ; E. MATVEEVA ; J.R. LAKOWICZ.** Topics in Fluorescence in Fluorescence Spectroscopy. Kluwer Academic/Plenum Publishers, 2005, 401-448
- **J.R. LAKOWICZ ; I. GRYCZYNSKI ; J. MALICKA ; Z. GRYCZYNSKI ; C.D. GEDDES.** Enhanced and localized multiphoton excited fluorescence near metallic silver islands: metallic islands can increase probe photostability. *J.Fluoresc.,* 2002, vol. 12, 299-302
- **C.D. GEDDES ; H. CAO ; I. GRYCZYNSKI ; Z. GRYCZYNSKI ; J. FANG ; J.R. LAKOWICZ.** Metal-enhanced fluorescence due to silver colloids on a planar surface: Potential applications of Indocyanine green to in vivo imaging. *J. Phys. Chem. A.,* 2003, vol. 107, 3443-3449
- **K. ASLAN ; J.R. LAKOWICZ ; C. D. GEDDES.** Rapid deposition of triangular silver nanoplates on planar surfaces: Application to metalenhanced fluorescence. *J. Phys. Chem. B.,* 2005, vol. 109, 6247-6251
- **K. ASLAN ; Z. LEONENKO ; J.R. LAKOWICZ ; C.D. GEDDES.** Fast and slow deposition of silver nanorods on planar surfaces: Application to metalenhanced fluorescence. *J. Phys. Chem. B.,* 2005, vol. 109 (8), 3157-3162
- **C.D. GEDDES ; A. PARFENOV ; D. ROLL ; J. FANG ; J. R. LAKOWICZ.** Electrochemical and laser deposition of silver, for use in metal enhanced fluorescence. *Langmuir,* 2003, vol. 19, 6236-6241

- **K. ASLAN ; R. BADUGU ; J.R. LAKOWICZ ; C.D. GEDDES.** Metal-enhanced fluorescence from plastic substrates. *J. Fluoresc.,* 2005, vol. 15 (2), 99-104
- **K. ASLAN ; C.D. GEDDES.** Microwave-Accelerated Metal-Enhanced Fluorescence (MAMEF): A New Platform Technology for Ultra Fast and Ultra Bright Assays. *Anal. Chem.,* vol. 77 (24), 8057-8067
- **J.R. LAKOWICZ.** Radiative decay engineering: Biophysical and biomedical applications. *Anal. Biochem.,* 2001, vol. 298, 1-24
- **J.R. LAKOWICZ ; C.D. GEDDES ; I. GRYCZYNSKI ; J. MALICKA ; Z. GRYCZYNSKI ; K. ASLAN ; J. LUKOMSKA ; E. MATVEEVA ; J. ZHANG ; R. BADUGU.** Advances in surface-enhanced. *fluorescence, J. Fluoresc.,* 2004, vol. 14 (4), 425-441
- **K. ASLAN ; Z. LEONENKO ; J.R. LAKOWICZ ; C.D. GEDDES.** Annealed Silver-Island Films for Applications in Metal-Enhanced Fluorescence: Interpretation in Terms of Radiating Plasmons. *J. of Fluores.,* 2005, vol. 15 (5), 643-654
- **I. GRYCZYNSKI ; J. MALICKA ; Z. GRYEZYNSKI ; J.R. LAKOWICZ.** Radiative Decay Engineering 4. Experimental studies of surface plasmon-coupled directional emission. *Anal. Biochem.,* 2004, vol. 324, 170-182
- **C.D. GEDDES ; I. GRYCZYNSKI ; J. MALICKA ; Z. GRYCZYNSKI ; J.R. LAKOWICZ.** Directional surface plasmon coupled emission. *J. Fluoresc.,* 2004, vol. 14, 119-123
- **J. YGUERABIDE ; W YGUERABIDE.** *Anal Biochem.,* 1998, vol. 262, 137-176
- **L. RIVAS ; S. SANCHEZ-CORTES ; J.V. GARCIA-RAMOS ; G. MORCILLO G.** Growth of Silver Colloidal Particles Obtained by Citrate Reduction to Increase the Ramen Enhancement Factor. *Langmuir,* 2001, vol. 17 (3), 574-577
- **N. SHIRTCLIFFE ; U. NICKEL ; S. SCHNEIDER.** Reproducible preparation of silver, sols with small particle size using borohydride reduction: For use as nuclei fox preparation of large particles. *J. Colloid Interface Sci.,* 1999, vol. 211 (1), 122-129
- **I. PASTORIZA-SANTOS ; L.M> LIZ-MARZAN.** Reduction of silver nanoparticles in DMF. Formation of monolayers and stable colloids. *Pure Appl. Chem.,* 2000, vol. 72 (1-2), 83-90
- **PASTORIZA-SANTOS, C. SERRA-RODRIQUEZ ; L.M. LIZ-MARZAN.** Self-assembly of silver particle monolayers on glass from Ag.sup.+ solutions in DMF. *J. Colloid Interface Sci.,* 2000, vol. 221 (2), 236-241
- **R. BRIGHT ; M.D. MUSICK ; MJ. NATAN.** Preparation and characterization of Ag colloid monolayers. *Langmuir,* 1998, vol. 14 (20), 5695-5701
- **F. NI ; T.M.** Chemical procedure for preparing surface-enhanced Roman scattering active silver films. *Anal. Chem.,* 1986, vol. 58 (14), 3159-5163
- **U. KRELBIG ; M. GARTZ ; A. HILGER.** Mie resonances: Sensors for physical and chemical cluster interface properties, Ber. Bunsenges. *Phys. Chem.,* 1997, vol. 101 (11), 1593-1604
- **R.G. FREEMAN ; K.C, GRABAR ; K.J. ALLISON ; R.M. BRIGHT R. ; J.A. DAVIS ; A.P. GUTHRIE ; M.B. HOMMER ; M.A. JACKSON ; P.C. SMITH ; D.G WALTER.** Self-assembled metal colloid monolayers: An approach to SERS substrate. *Science,* 1995, vol. 267, 1629-1632
- **K.C. GRABAR ; R.G FREEMAN ; M.B. HOMMER ; M.J. NATAN.** Preparation and characterization of Au colloid monolayers. *Anal. Chem.,* 1995, vol. 67, 735-743
- **LAKOWICZ, J. R.** *Anal. Biochem.,* 2004, vol. 324, 153-169
- **ASLAN, K. ; PÉREZ-LUNA, V. H.** *Langmuir,* 2002, vol. 18, 6059-6065
- **CORMIER, M. J. ; PRICHARD, P. M.** *J Biol. Chem.,* vol. 106.8 (243), 4706-4714
- Methods in Enzymology. 1978, vol. VLII